# EUROPEAN PATENT SPECIFICATION

(11) **EP 3 743 436 B1**
(45) Date of publication and mention of the grant of the patent: **26.08.2026**
(21) Application number: 19743421.0
(22) Date of filing: 23.01.2019
(51) Int. Cl.: A61K 39/12, A61K 35/17, A61K 38/00, A61K 35/15, C07K 16/084, A61K 38/17, A61P 31/20, A61K 39/00, C12N 5/0783, C12N 5/078, C07K 14/005, A61P 35/00

(54) **HPV IMMUNOTHERAPY**
HPV-IMMUNTHERAPIE
IMMUNOTHÉRAPIE DE L'HPV

(30) Priority: 24.01.2018 US 201862621279 P
(43) Date of publication of application: 02.12.2020
(73) Proprietor: The Council of the Queensland Institute of Medical Research, Herston, Queensland 4006 (AU)
(72) Inventor: KHANNA, Rajiv, Herston, QLD 4006 (AU)
(74) Representative: Vossius & Partner Patentanwälte Rechtsanwälte mbB
(86) International application number: PCT/IB2019/000131
(87) International publication number: WO 2019/145792

(56) References cited:
- WO-A1-2008/145685
- WO-A1-2013/105856
- WO-A1-2016/146618
- WO-A1-2017/220463
- WO-A1-2019/173465
- WO-A1-91/18294
- WO-A2-00/75336
- WO-A2-02/090382
- WO-A2-2004/011650
- WO-A2-2004/098497
- WO-A2-2005/089164
- WO-A2-2015/086354
- US-A- 6 037 135
- US-A1- 2004 147 044
- US-A1- 2005 142 541
- US-A1- 2006 182 763
- MARYAM DADAR ET AL: "Advances in Designing and Developing Vaccines, Drugs and Therapeutic Approaches to Counter Human Papilloma Virus", FRONTIERS IN IMMUNOLOGY, vol. 9, 12 November 2018 (2018-11-12), XP055604482, ISSN: 1664-3224, DOI: 10.3389/fimmu.2018.02478
- DILLNER J.: "Mapping of linear epitopes of human papillomavirus type 16: The El, E2, E4, E5, E6 and E7 open reading frames", INTERNATIONAL JOURNAL OF CANCER, vol. 46, no. 4, 1990, pages 703 - 711, XP000576502, DOI: 10.1002/ijc.2910460426
- ERIKSSON A. ET AL.: "Human papillomavirus type 16 variant lineages characterized by nucleotide sequence analysis of the E5 coding segment and the E2 hinge region", JOURNAL OF GENERAL VIROLOGY, vol. 80, no. 3, 1999, pages 595 - 600, XP055627154
- NEZAFAT N. ET AL.: "A novel multi-epitope peptide vaccine against cancer: An in silico approach", JOURNAL OF THEORETICAL BIOLOGY, vol. 349, 2014, pages 121 - 134, XP028835060, DOI: 10.1016/j.jtbi.2014.01.018

## Description

### BACKGROUND

Human papillomaviruses (HPVs) are a family of viruses comprising more than 150 types. Selective infection of skin or mucous membranes is a classic feature of HPVs, and their replication is closely linked to the maturation of the cells in these membranes. The most common HPV types are the low-risk HPV-6 and HPV-11, which are responsible for 90% of genital warts and a disease known as recurrent respiratory papillomatosis, in which tumors grow in the airway. HPV-16 and HPV-18 account for the majority of cancers of the cervix, anus, vagina, vulva, penis, tongue base, larynx, and tonsil. HPV plays a role in the development of non-melanoma skin cancer (NMSC), including cutaneous squamous cell carcinoma (SCC), among chronic lymphocytic leukemia (CLL) and blood and marrow transplant (BMT) patients. Recent United States population-based studies conducted by the Centre for Disease Control show that 66% of cervical cancers, 55% of vaginal cancers, 79% of anal cancers, and 62% of oropharyngeal cancers are attributable to HPV types 16 or 18. Globally, HPV infection accounts for an estimated 530,000 cervical cancer cases (~270,000 deaths) annually, with the majority (86% of cases, 88% of deaths) occurring in developing countries. In total, HPV accounts for 5.2% of the worldwide cancer burden. Each year in the United States, an estimated 26,000 new cancers are attributable to HPV, about 17,000 in women and 9,000 in men.

Standard treatment options for HPV-associated lesions include physical elimination by cryotherapy (i.e. using extreme cold to destroy tissue), chemical cauterization (i.e. using a chemical to destroy tissue), and laser or surgical removal. However, in pre-cancerous lesions, surgical procedures alone are not very effective, since 20-30% or more cases recur, with lesions both at previously treated sites due to failure of the procedure to eliminate the HPV, and at new sites due to new infections. When this occurs, radiotherapy and chemotherapy are then used with relative success; however, about 50% of the HPV-associated cancer patients still die of the disease. Clearly, new treatment strategies are urgently needed to control the burden of HPV-related cancer.

### SUMMARY OF THE INVENTION

The present invention is defined by the claims. Accordingly, the present invention relates to a vaccine comprising a viral vector encoding polyepitope peptides comprising Human Papillomavirus (HPV) peptide epitopes capable of inducing proliferation of peptide-specific T cells and a pharmaceutically acceptable carrier, diluent or excipient, wherein the viral vector is a recombinant adenovirus Ad5F35 vector, and wherein the vector encodes the CD8polyIRESCD4poly sequence of 
ATGTTCGAGCTGTCGCAAATGGTGCAGTGGACCCTGCTGCAACAGTACTGC
CTGTACTTGTCCGAGATCGCTTACAAATACGCCCAGCGGCCCTTTAAGAGC
AACAAGTCAACCACCCTGCAAGACGTCAGCCTCGAAGTGTACCTCGTGTGG
GAAGTCCACGCGGGTGGACAGGTCATCTGGCCCACAACTCCTCCGAGGCCA
ATCAATCTGGATACGGCGTCCACTACCCTCTCCGCCTTCCGGTGCTTCATCG
TGTACTCCCCCTGCCACTGCCTTCACCGTGTATCACGACATTATTCTGGAATG
CGTGTACAAGCTCCCGCAGCTTTGTACCGAGCTGTTCCGCGATCTGTGCAT
CGTGTACCGCATTCGGTGCATCAACTGTCAGAAGCCGCTGACTGTGCTGGA
GCTGACCGAAGTGTTTGAGTTCGCCCTGTTGATGGGCACCCTTGGGATTGT
GGACAGAGCACATTACAACATCGTCACCTTCTGATAATAAGGTCTCTAAAAT
TCCGCCCCCCCCCTAACGTTACTGGCCGAAGCCGCTTGGAATAAGGCCGGTGTG
CGTTTGTCTATATGTTATTTTCCACCATATTGCCGTCTTTTGGCAATGTGAGGGCC
CGGAAACCTGGCCCTGTCTTCTTGACGAGCATTCCTAGGGGTCTTTCCCCTCTCG
CCAAAGGAATGCAAGGTCTGTTGAATGTCGTGAAGGAAGCAGTTCCTCTGGAAG
CTTCTTGAAGACAAACAACGTCTGTAGCGACCCTTTGCAGGCAGCGGAACCCCC
CACCTGGCGACAGGTGCCTCTGCGGCCAAAAGCCACGTGTATAAGATACACCTG
CAAAGGCGGCACAACCCCAGTGCCACGTTGTGAGTTGGATAGTTGTGGAAAGAG
TCAAATGGCTCTCCTCAAGCGTATTCAACAAGGGGCTGAAGGATGCCCAGAAGG
TACCCCATTGTATGGGATCTGATCTGGGGCCTCGGTACACATGCTTTACATGTGT
TTAGTCGAGGTTAAAAAACGTCTAGGCCCCCCGAACCACGGGGACGTGGTTTTC
CTTTGAAAAACACGATGATAATATGGCCACAACCATGCGCTACATGATTCTGGGG
CTCCTTGCACTCGCGGCCGTGTGCTCAGCCTGGAAGTCCTTTTTCTCCCGGACTTGGT
CGAGACTGTCACTCCATCATATCGACTACTGGAAGCACATGCGCCTGGAATGCGCCCT
GTACTCGGTGGATTCCGCCCCGATCCTGACCGCTTTCAACTCCTCCCACGTCTATGAC
TACGCCTTCCGGGACCTCTGTATTGTCTACCGGGATGGGAACCCGTACGCCGTGTGT
GACCGGTGCATCAATTGCCAGAAGCCACTCTGCCCTGAGGAAAAGCAGAGGCATCTG
GACGAGAAGCAACGCCATTTGGATAAGAAACAGCGGTTCCACAACATCAGAGGTCGC
TGGACTGGCCGGTGCATGTCCTGCTGCACCCCCACCCTGCACGAGTACATGCTGGAT
CTGCAGCCCGAGACTACCGACCTGTACTACGAACAGCTTAACGACAGCTCCGAGGAA
GAGGACGAAATCGACGTGCAAGCACCCACGTGGACATCAGGACCCTGCAAGATCTC
CTGATGGGCACCCTGACGTTGATCCCTATTGCTGTCGGTGGAGCACTTGCCGGCCTG
GTGCTGATCGTGCTGATCGCCTATCTCGTGGGACGCAAGAGAAGCCACGCGGGATAC
CAGACTATTTGATAA
 HPV CD8poly
IRES
*HPV CD4poly*

In accordance with a preferred embodiment the immunogenic peptides are capable of inducing proliferation of peptide-specific cytotoxic T cells (CTLs).

The present invention also relates to the vaccine of the invention for use in treating and/or preventing a cancer expressing an HPV epitope or a precancerous condition expressing an HPV epitope in a subject and for use in treating and/or preventing an HPV infection in a subject.

### BRIEF DESCRIPTION OF THE DRAWINGS

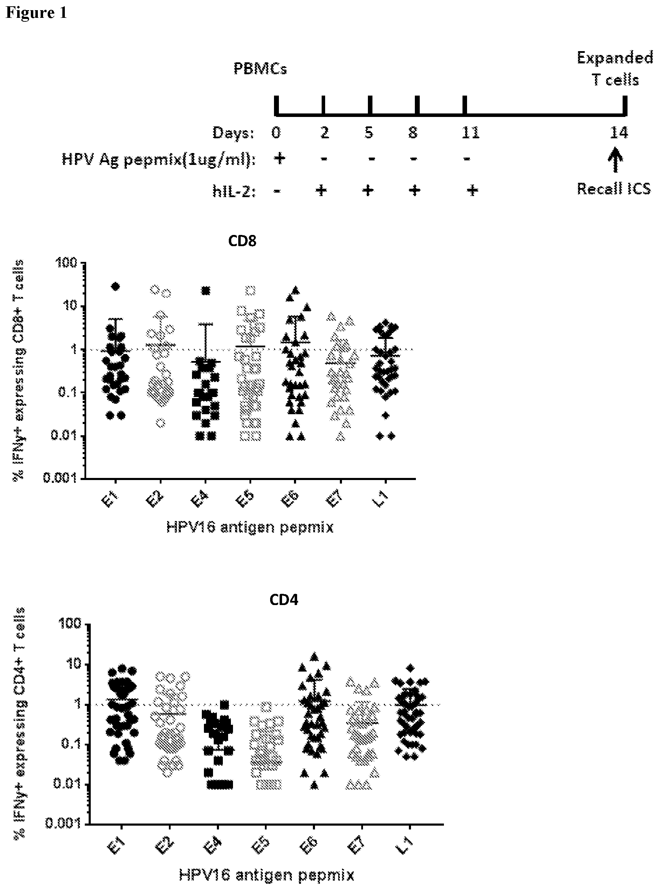

**Figure 1** shows the overall T cell response to HPV16 antigens in HNC patients.

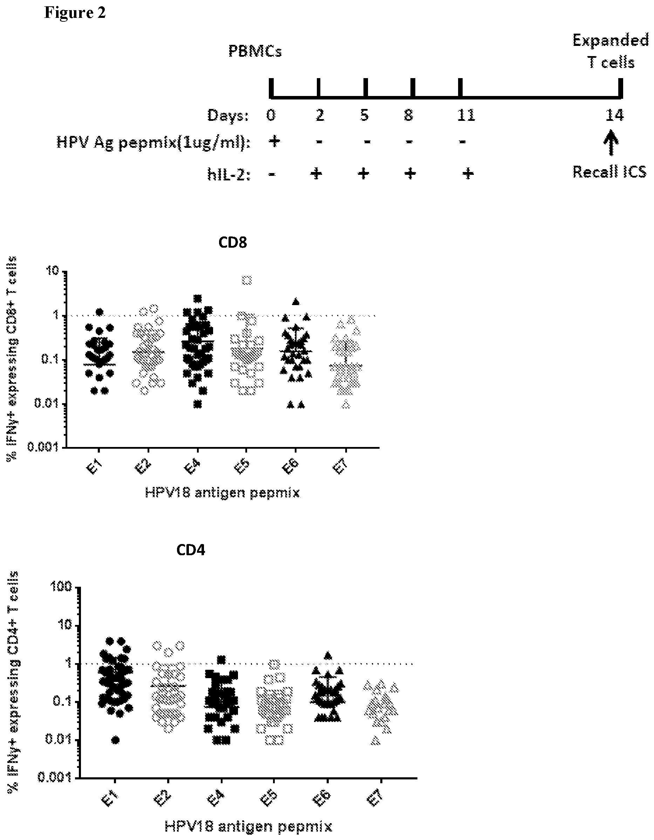

**Figure 2** shows the overall T cell response to HPV18 antigens in HNC patients.

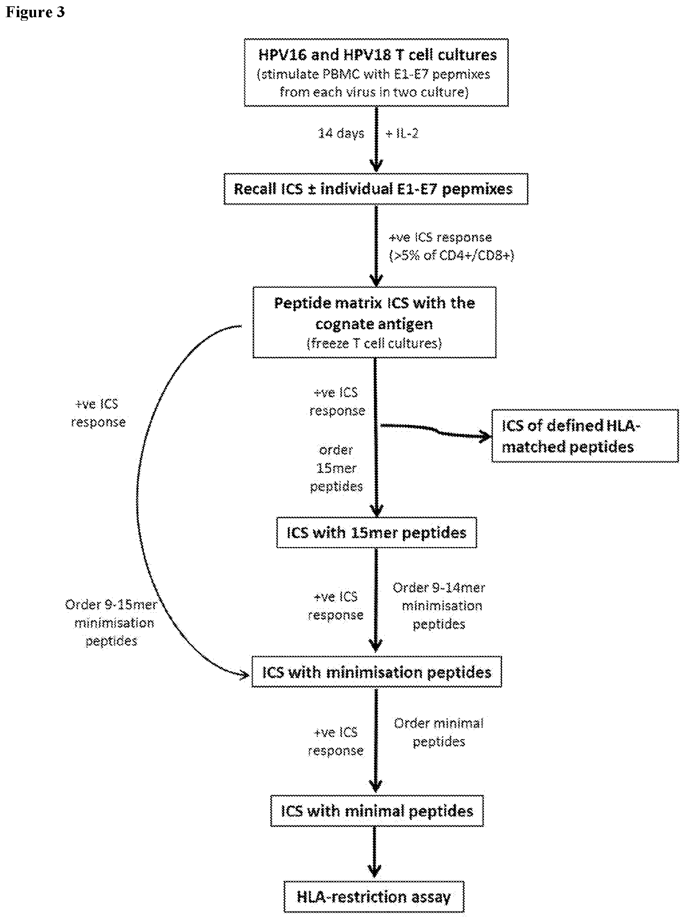

**Figure 3** is a flow chart showing the process of epitope mapping described herein.

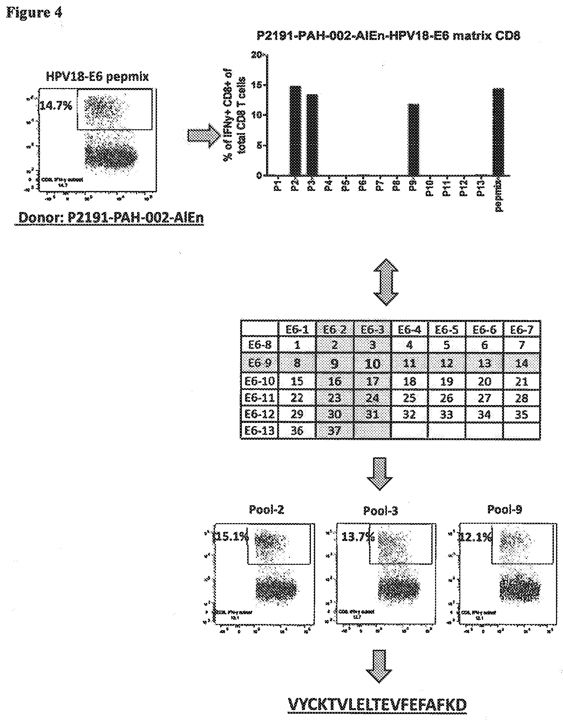

**Figure 4** shows representative work flow strategy for identifying HPV epitope sequences.

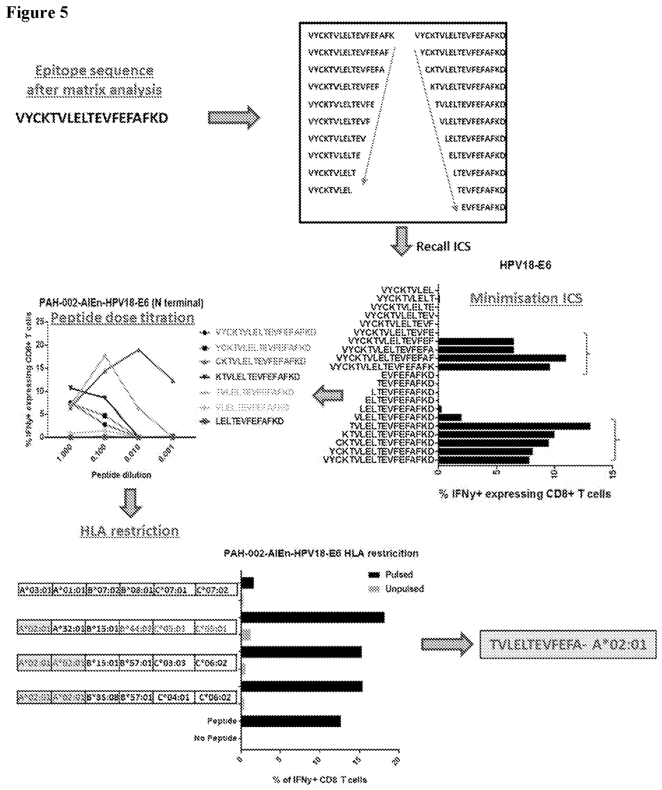

**Figure 5** illustrates the fine-mapping process that identified HPV18-E6 CD8⁺ T cell epitope TVLELTEVFEFA.

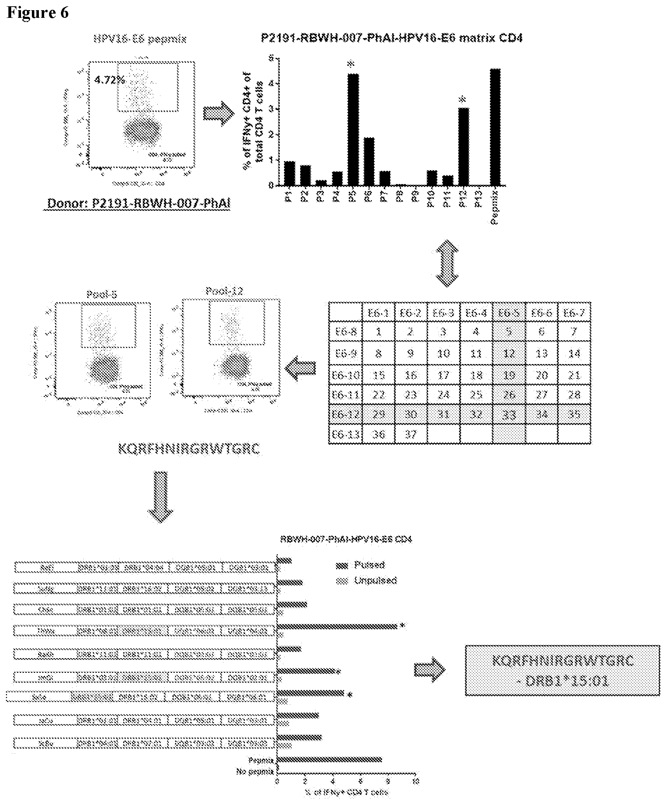

**Figure 6** illustrates the fine CD4+ T cell epitope-mapping process of HPV16-E6 pools that identified epitope KQRFHNIRGRWTGRC.

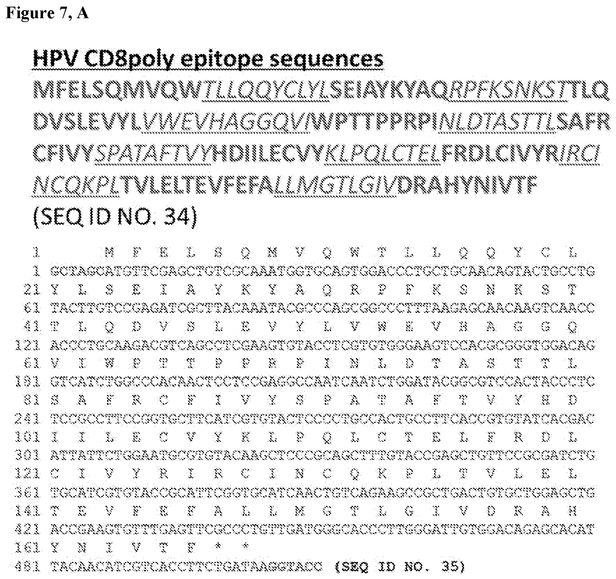

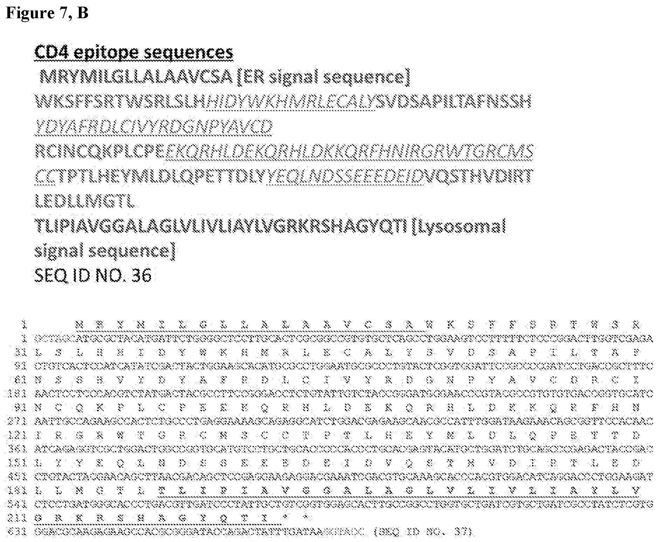

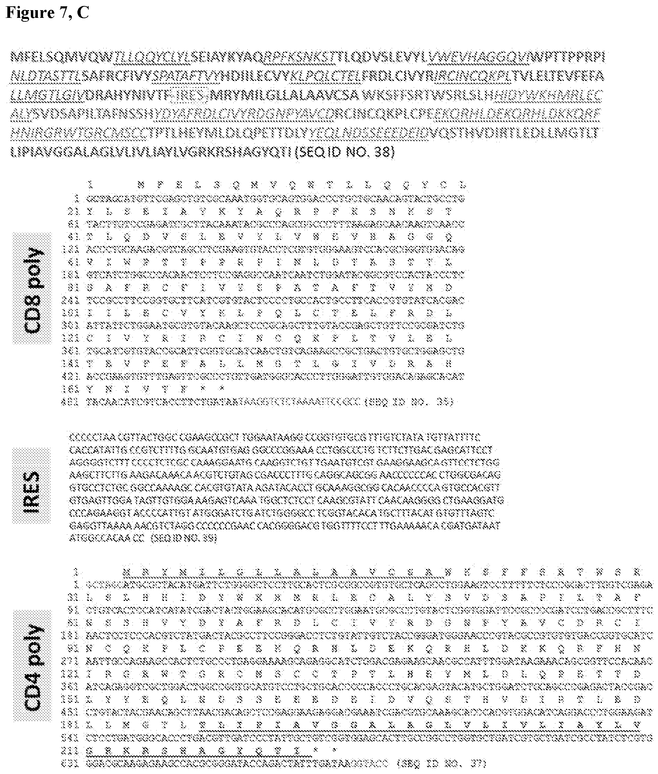

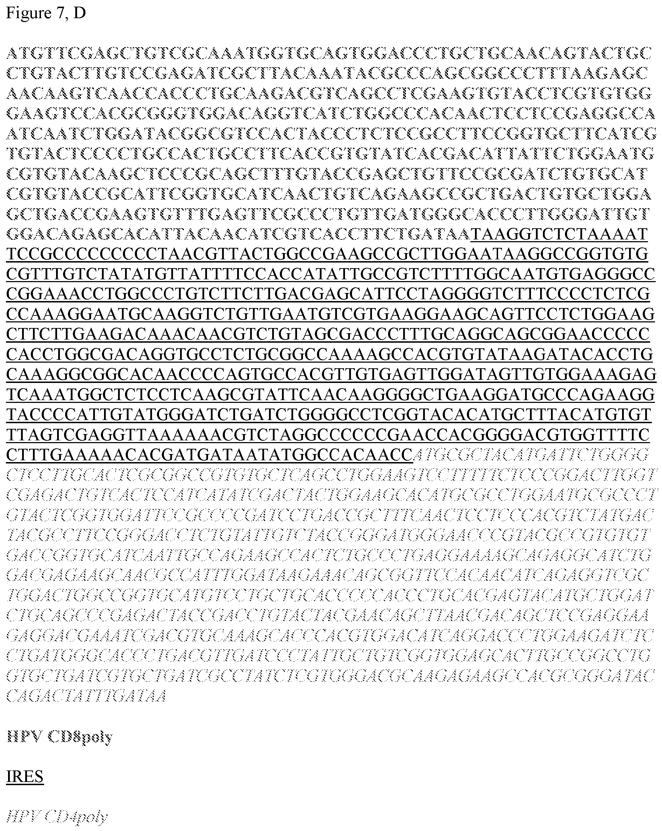

**Figure 7****, A-D** shows schematic design of the HPV polyepitope protein constructs encoding CD8⁺ T cell polyepitope, CD4⁺ T cell polyepitope and CD8IRESCD4 polyepitope. Each of the T cell epitope sequences (selected fron Table 1) are shown in alternating bold and underlined italic text. The HPV CD4⁺ T cell polyepitope includes an ER (endoplasmic reticulum) signal sequence at the amino-terminus and a lysosomal signal sequence at the carboxy-terminus (B). For making CD8⁺ and CD4⁺ T cell polyepitope construct, both the polyepitope sequences were separated by an internal ribosome entry site (IRES) sequence derived from encephalomyocarditis virus (C, amino acid sequence of translated peptides; and D, nucleic acid sequence encoding CD8IRESCD4 polyepitope polypeptides, wherein CD8 sequence is shown in bold, IRES sequence is underlined, and CD4 sequence is shown in italics). The DNA sequence encoding the CD8^{+,} CD4⁺ and CD8⁺ and CD4⁺ T cell epolyepitopes were obtained from Atum Bio in pJ201 vector.

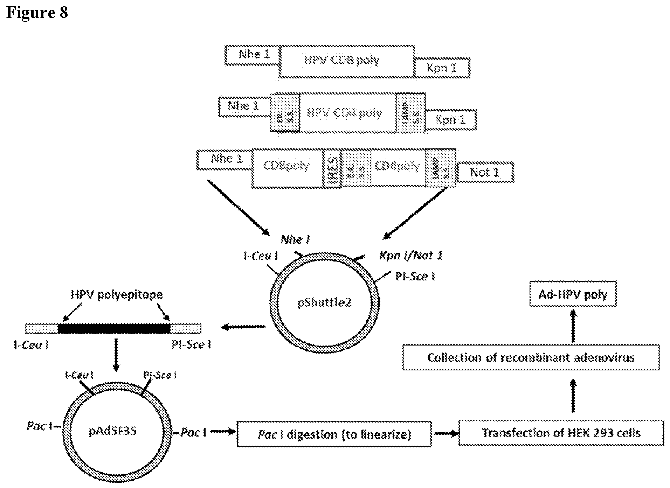

**Figure 8** shows the outline of the cloning of HPV polyepitope protein constructs encoding CD8+ T cell polyepitope, CD4⁺ T cell polyepitope, and CD8IRESCD4 T cell polyepitope into Ad5F35 vector. DNA sequence encoding the HPV CD8⁺, CD4⁺ or CD8IRESCD4 T cell polyepitopes was cloned into a pShuttle2 vector using restriction sites specified in the figure and then subcloned into the Ad5F35 expression vector. Recombinant Ad5F35 virus expressing HPV CD8⁺, CD4⁺ or CD8IRESCD4 T cell polyepitopes was made by infecting HEK293 cells with Ad5F35 vector. The recombinant adenovirus was harvested from transfected HEK293 cells by repeated freeze-thawing cycles.

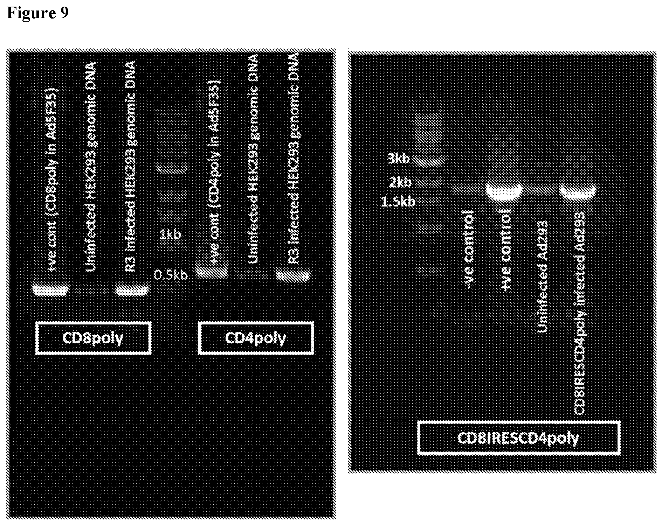

**Figure 9** shows PCR confirmation of AdHPVCD8, AdHPVCD4 and AdCD8IRESCD4 polyepitope recombinant virus master stock. HEK293 cells were infected with recombinant adenovirus encoding HPV CD8⁺, CD4⁺ or CD8IRESCD4 polyepitopes. Genomic DNA was isolated and subjected to PCR. DNA from uninfected HEK293 cells or adenovirus vector encoding CD8⁺, CD4⁺ or CD8IRESCD4 polyepitopes were used as negative and positive controls, respectively.

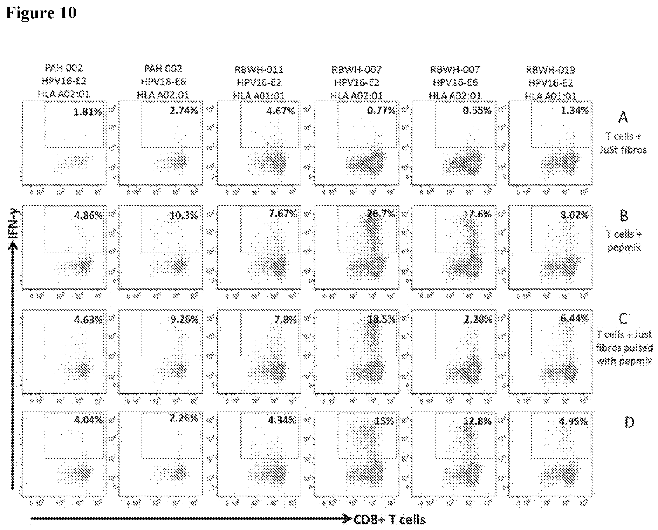

**Figure 10** shows FACS plots of presentation of AdHPVCD8polyepitope by JuSt fibroblast cells. JuSt fibroblasts cells were activated with IFN-γ 24 hours prior to assay and then pulsed with AdHPVCD8poly recombinant virus for one hour. Cells were then washed, incubated overnight, and then exposed to HPV-specific CD8⁺ T cells specific for various HLA restricted (HLA A02:01 and HLA A01:01) epitopes obtained from different HPV antigens (HPV16-E2, HPV18-E6, HPV16-E6). FACS analysis shows IFN-γ expression by the HPV-specific CD8⁺ T cells following co-culture with AdHPVCD8poly or HPV pepmix (positive control) pulsed JuSt fibroblast cells.

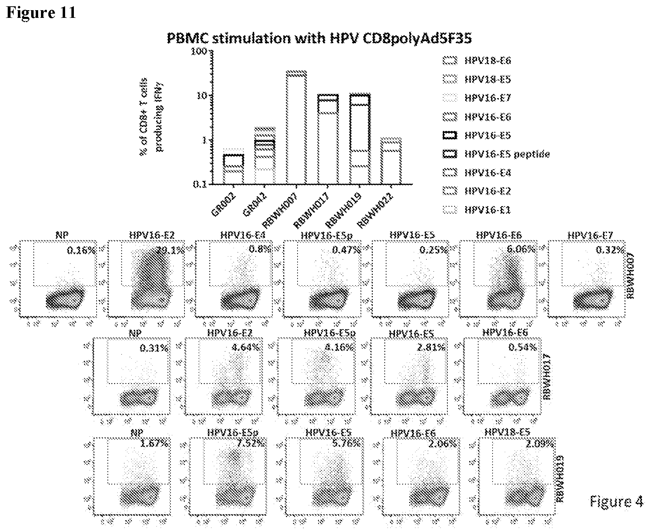

**Figure 11** shows expansion of HPV-specific CD8⁺ T cells from HPV HNC patients. PBMC (5 X 10⁶) was stimulated with HPV AdCD8poly recombinant virus and cultured for 14 days in the presence of IL-2. The frequency of HPV-specific CD8+ T cells was determined by measuring IFN-γ secretion in response to stimulation with HPV-specific pepmix. Staked bar graphs and representative FACS plots showing percentage of HPV-specific CD8+ T cells expressing IFN-γ.

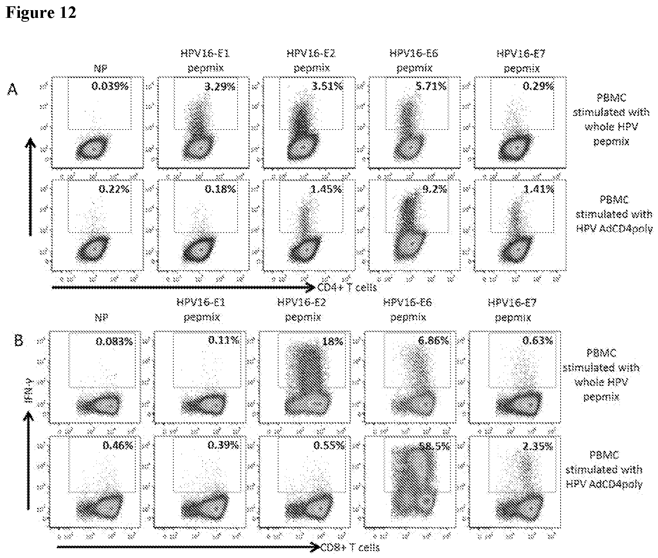

**Figure 12** shows expansion of HPV-specific CD4⁺ and CD8⁺ T cells from an HPV HNC patient. PBMC (5 X 10⁶) was stimulated with HPV AdCD4poly recombinant virus and cultured for 14 days in the presence of IL-2. In the same experiment PBMC was stimulated with HPV pepmix as a comparison control. The frequency of HPV-specific CD4⁺ and CD8⁺ T cells were determined by measuring IFNγ secretion in response to stimulation with HPV-specific pepmix.

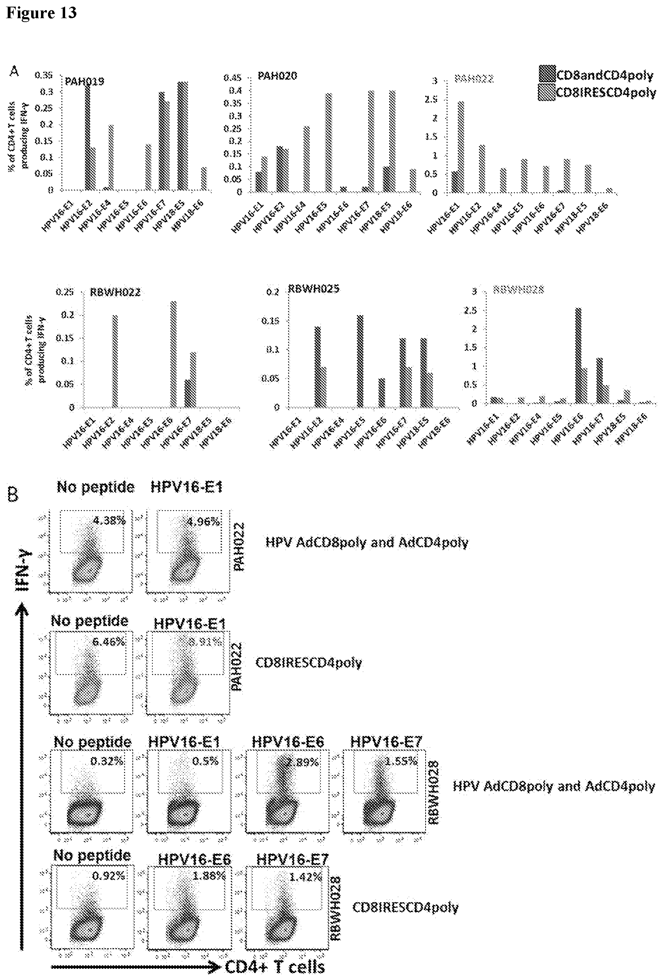

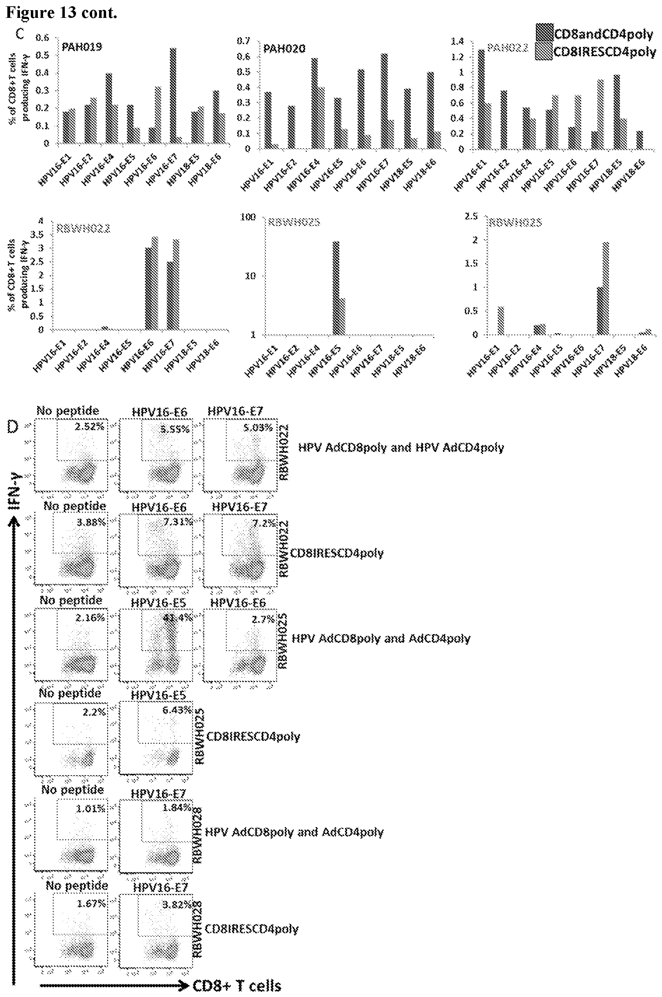

**Figure 13****, A-D** shows expansion of HPV-specific CD8⁺ and CD4⁺ T cells from HPV HNC patients. PBMC (5 X 10⁶) from HPV HNC patients were stimulated with HPV AdCD8IRESCD4poly recombinant virus and cultured for 14 days in the presence of IL-2. As a comparison controls PBMC stimulated with HPV AdCD8poly and AdCD4poly also included in the same experiment. The frequency of HPV-specific CD4⁺ (Panel A & B) and CD8⁺ (Panel C & D) T cells were determined by measuring IFN-γ secretion in response to stimulation with HPV-specific pepmix. Bar graphs and representative FACS plots showing percentage of HPV-specific CD8⁺ T cells expressing IFN-γ.

### DETAILED DESCRIPTION

### General

The references to the methods of treatment by therapy or surgery or in vivo diagnosis methods in this detailed description are to be interpreted as references to compounds, pharmaceutical compositions and medicaments of the present disclosure for use in those methods. While innate immune responses play an important role in controlling initial HPV infection, long-term protection is dependent on adaptive immune responses including humoral and cell-mediated immunity. In immunocompetent individuals, the majority of HPV infections are cleared within 2 years of initial infection. Infiltration of CD4⁺ and CD8⁺ T cells is frequently observed in spontaneously regressing lesions.

A number of immunotherapeutic strategies have been tested for the treatment of HPV-associated diseases. While the HPV prophylactic vaccine is based on L1 protein, this viral antigen is not relevant for the treatment of HPV-associated diseases. This protein is only expressed in the late stages of HPV replication especially in terminally differentiated keratinocytes. In contrast, other proteins associated with the HPV replicative cycle, i.e., E1, E2, E6 and E7, have been identified as important targets for immunotherapeutic strategies. This is primarily due to the fact that the expression of all these proteins are retained through multiple stages of infection. While much of the emphasis on the design of immunotherapeutic strategies has focused on E6 and E7 antigens, it is important to appreciate that E1 and E2 proteins are implicated in HPV DNA replication and thus the expression of these proteins is retained throughout multiple stages of infection. This highlights the importance of these proteins as potential targets for immunotherapy aimed at eliminating persistently HPV-infected cells regardless of the stage of pathogenesis. Indeed, previous studies using animal models (canine and rabbit) have shown that immunisation with a DNA vaccine encoding codon-optimised E1 or E2 genes results in complete regression of papillomas. The primary mode of protection in these animal models is mediated through the induction of an effective T cell response to E1 and E2 antigens. Further clinical studies using a modified vaccinia Ankara vector encoding E2 in human subjects with HPV-induced cervical lesions (C1N1 to C1N3) demonstrated complete elimination of cervical lesions to regression from C1N3 to C1N1 and significant reduction in HPV viral load. Here again, the induction of E2-specific T cell immunity correlated strongly with clinical response. Development of anti-vector antibodies resulted in a poor response to booster immunisation and some patients showed recurrence of lesions after the completion of the study. Moreover, this therapy required direct injection of the vector into uterine tissue to be effective, thus limiting its wider use in the general population.

Retrospective clinical studies have been done on patients who had high-grade intraepithelial lesions that regressed to cleared cervical intraepithelial neoplasia lesions, and subsequently exhibited reduced HPV viral load. HLA class II-restricted CD4⁺ T cell epitopes from HPV E7 protein were identified in these patients, and immune profiling of their peripheral blood mononuclear cells revealed that the HPV E7-specific T cells displayed a Th1 bias, characterised by IFN-γ and TNF expression. Indeed, ex vivo analysis in patients showing regression of HPV-driven pathology post-intervention without disease recurrence revealed that a strong T cell response was directed towards the E6 and E7 proteins. In contrast, T cells from patients who were diagnosed with recurrent disease did not show this antigen specificity profile. Reconstitution of robust T cell immunity against the E6 and E7 antigens may help confer long-term protection from disease recurrence.

Provided herein are compositions and methods related to HPV epitopes (*e.g*., HPV epitopes listed in Table 1) that are recognized by cytotoxic T lymphocytes (CTLs) and that are useful in the prevention and/or treatment of HPV infection, and/or cancer, and/or precancerous lesions. In certain aspects, provided herein are compositions (*e.g*., prophylactic and/or therapeutic compositions, such as vaccine compositions) containing a polypeptide comprising one or more of the HPV epitopes described herein (*e.g.,* HPV epitopes listed in Table 1), nucleic acids encoding such a polypeptide, CTLs that recognize such a peptide, APCs presenting such peptides and/or antigen-binding molecules that bind specifically to such peptides, as well as methods of treating and/or preventing HPV infection, and/or cancer, and/or precancerous lesions by administering such compositions to a subject. In some implementations, also provided herein are methods of identifying a subject suitable for treatment according to a method provided herein.

### Definitions

For convenience, certain terms employed in the specification, examples, and appended claims are collected here.

The articles "*a*" and "*an*" are used herein to refer to one or to more than one (*i.e.,* to at least one) of the grammatical object of the article. By way of example, "an element" means one element or more than one element.

As used herein, the term *"administering"* means providing a pharmaceutical agent or composition to a subject, and includes, but is not limited to, administering by a medical professional and self-administering. Such an agent can contain, for example, peptide described herein, an antigen presenting cell provided herein and/or a CTL provided herein.

The term *"amino acid"* is intended to embrace all molecules, whether natural or synthetic, which include both an amino functionality and an acid functionality and capable of being included in a polymer of naturally-occurring amino acids. Exemplary amino acids include naturally-occurring amino acids; analogs, derivatives and congeners thereof; amino acid analogs having variant side chains; and all stereoisomers of any of the foregoing.

As used herein, the term *"antibody"* may refer to both an intact antibody and an antigen binding fragment thereof. Intact antibodies are glycoproteins that include at least two heavy (H) chains and two light (L) chains inter-connected by disulfide bonds. Each heavy chain includes a heavy chain variable region (abbreviated herein as V_{H}) and a heavy chain constant region. Each light chain includes a light chain variable region (abbreviated herein as V_{L}) and a light chain constant region. The V_{H} and V_{L} regions can be further subdivided into regions of hypervariability, termed complementarity determining regions (CDR), interspersed with regions that are more conserved, termed framework regions (FR). The variable regions of the heavy and light chains contain a binding domain that interacts with an antigen. The constant regions of the antibodies may mediate the binding of the immunoglobulin to host tissues or factors, including various cells of the immune system (*e.g.,* effector cells) and the first component (Clq) of the classical complement system. The term "antibody" includes, for example, monoclonal antibodies, polyclonal antibodies, chimeric antibodies, humanized antibodies, human antibodies, multispecific antibodies (*e.g*., bispecific antibodies), single-chain antibodies and antigen-binding antibody fragments.

The terms *"antigen-binding fragment"* and *"antigen-binding portion"* of an antibody, as used herein, refers to one or more fragments of an antibody that retain the ability to bind to an antigen. Examples of binding fragments encompassed within the term "antigen-binding fragment" of an antibody include Fab, Fab', F(ab')₂, Fv, scFv, disulfide linked Fv, Fd, diabodies, single-chain antibodies, camelid antibodies, isolated CDRH3, a Designed Ankyrin Repeat Protein (DARPin) and other antibody fragments that retain at least a portion of the variable region of an intact antibody. These antibody fragments can be obtained using conventional recombinant and/or enzymatic techniques and can be screened for antigen binding in the same manner as intact antibodies.

The term *"binding"* or *"interacting"* refers to an association, which may be a stable association, between two molecules, e.g., between a peptide and a binding partner or agent, *e.g.*, small molecule, due to, for example, electrostatic, hydrophobic, ionic and/or hydrogen-bond interactions under physiological conditions.

The term *"biological sample*," *"tissue sample,"* or simply *"sample"* each refers to a collection of cells obtained from a tissue of a subject. The source of the tissue sample may be solid tissue, as from a fresh, frozen and/or preserved organ, tissue sample, biopsy, or aspirate; blood or any blood constituents, serum, blood; bodily fluids such as cerebral spinal fluid, amniotic fluid, peritoneal fluid or interstitial fluid, urine, saliva, stool, tears; or cells from any time in gestation or development of the subject.

As used herein, the term *"cancer"* includes, but is not limited to, solid tumors and blood borne tumors. The term cancer includes diseases of the skin, tissues, organs, bone, cartilage, blood, and vessels, including the cervix, anus, vagina, vulva, penis, tongue base, larynx, and tonsil. The term "cancer" further encompasses primary and metastatic cancers.

The term "precancerous lesions" or "precancerous condition" refers to atypical cells and/or tissues that are associated with an increased risk of cancer. The term "precancerous lesions" may refer, for example, to dysplasia, benign neoplasia, or carcinoma in situ.

The term *"epitope"* means a protein determinant capable of specific binding to an antibody. Epitopes usually consist of chemically active surface groupings of molecules such as amino acids or sugar side chains. Certain epitopes can be defined by a particular sequence of amino acids to which a T cell receptor or antibody is capable of binding.

The term *"isolated nucleic acid"* refers to a polynucleotide of natural or synthetic origin or some combination thereof, which (1) is not associated with the cell in which the "isolated nucleic acid" is found in nature, and/or (2) is operably linked to a polynucleotide to which it is not linked in nature.

The term *"isolated polypeptide"* refers to a polypeptide, in certain implementations prepared from recombinant DNA or RNA, or of synthetic origin, or some combination thereof, which (1) is not associated with proteins that it is normally found with in nature, (2) is isolated from the cell in which it normally occurs, (3) is isolated free of other proteins from the same cellular source, (4) is expressed by a cell from a different species, or (5) does not occur in nature.

As used herein, the phrase *"pharmaceutically acceptable"* refers to those agents, compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, the phrase *"pharmaceutically-acceptable carrier"* means a pharmaceutically-acceptable material, composition or vehicle, such as a liquid or solid filler, diluent, excipient, or solvent encapsulating material, involved in carrying or transporting an agent from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; and (22) other non-toxic compatible substances employed in pharmaceutical formulations.

The terms *"polynucleotide",* and *"nucleic acid"* are used interchangeably. They refer to a polymeric form of nucleotides of any length, either deoxyribonucleotides or ribonucleotides, or analogs thereof. Polynucleotides may have any three-dimensional structure, and may perform any function. The following are non-limiting examples of polynucleotides: coding or non-coding regions of a gene or gene fragment, loci (locus) defined from linkage analysis, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. A polynucleotide may be further modified, such as by conjugation with a labeling component. In all nucleic acid sequences provided herein, U nucleotides are interchangeable with T nucleotides.

As used herein, a therapeutic that *"prevents"* a condition refers to a compound that, when administered to a statistical sample prior to the onset of the disorder or condition, reduces the occurrence of the disorder or condition in the treated sample relative to an untreated control sample, or delays the onset or reduces the severity of one or more symptoms of the disorder or condition relative to the untreated control sample.

As used herein, *"specific binding"* refers to the ability of an antibody to bind to a predetermined antigen or the ability of a peptide to bind to its predetermined binding partner. Typically, an antibody or peptide specifically binds to its predetermined antigen or binding partner with an affinity corresponding to a K_{D} of about 10⁻⁷ M or less, and binds to the predetermined antigen/binding partner with an affinity (as expressed by K_{D}) that is at least 10 fold less, at least 100 fold less or at least 1000 fold less than its affinity for binding to a non-specific and unrelated antigen/binding partner (*e.g*., BSA, casein).

As used herein, the term *"subject"* means a human or non-human animal selected for treatment or therapy.

The phrases "*therapeutically-effective amount"* and "*effective amount"* as used herein means the amount of an agent which is effective for producing the desired prophylactic and/or therapeutic effect in at least a sub-population of cells in a subject at a reasonable benefit/risk ratio applicable to any medical treatment.

*"Treating"* a disease in a subject or *"treating"* a subject having a disease refers to subjecting the subject to a pharmaceutical treatment, *e.g.,* the administration of a drug, such that at least one symptom of the disease is decreased or prevented from worsening.

The term *"vector"* refers to the means by which a nucleic acid can be propagated and/or transferred between organisms, cells, or cellular components. Vectors include plasmids, viruses, bacteriophage, pro-viruses, phagemids, transposons, and artificial chromosomes, and the like, that may or may not be able to replicate autonomously or integrate into a chromosome of a host cell.

### Peptides

Provided herein are peptides comprising HPV epitopes that are recognized by cytotoxic T lymphocytes (CTLs) and that are useful in the prevention and/or treatment of HPV infection, and/or cancer (*e.g.,* a cancer expressing an HPV epitope provided herein), and/or precancerous lesions. In certain implementations, the HPV epitope is an epitope listed in Table 1.

**Table 1. Exemplary HPV epitopes**

| **T cell specificity** | **Epitope** | **SEQ ID NO.:** | **HPV antigen** | **HLA restriction** |
|---|---|---|---|---|
| CD8⁺ | LQDVSLEVYL | 1 | HPV16-E2 | A*02:01 |
| CD8⁺ | TVLELTEVFEF | 2 | HPV18-E6 | A*02:01 |
| CD8⁺ | SPATAFTVY | 3 | HPV18-E5 | B*35:01 |
| CD8⁺ | SAFRCFIVY | 4 | HPV16-E5 | B*35:01 |
| CD8⁺ | FELSQMVQW | 5 | HPV16-E1 | B*18:01, B*44:02 |
| CD8⁺ | TLLQQYCLYL | 6 | HPV16-E1 | A*02:01 |
| CD8⁺ | SEIAYKYAQ | 7 | HPV16-E1 | B*18:01 |
| CD8⁺ | RPFKSNKST | 8 | HPV16-E1 | B*07:02 |
| CD8⁺ | TLQDVSLEVYL | 9 | HPV16-E2 | A*02:01 |
| CD8⁺ | VWEVHAGGQVI | 10 | HPV16-E2 | A*01:01 |
| CD8⁺ | WPTTPPRPI | 11 | HPV16-E4 | B*07:02 |
| CD8⁺ | NLDTASTTL | 12 | HPV16-E5 | C*05:01, C*08:02 |
| CD8⁺ | HDIILECVY | 13 | HPV16-E6 | B*18:01 |
| CD8⁺ | KLPQLCTEL | 14 | HPV16-E6 | A*02:01 |
| CD8⁺ | TIHDIILECV | 15 | HPV16-E6 | A*02:01 |
| CD8⁺ | FRDLCIVYR | 16 | HPV16-E6 | C*07:02 |
| CD8⁺ | AFRDLCIVY | 17 | HPV16-E6 | C*07:02 |
| CD8⁺ | IRCINCQKPL | 18 | HPV16-E6 | B*27:05 |
| CD8⁺ | GRWTGRCMSC | 19 | HPV16-E6 | B*27:05 |
| CD8⁺ | TVLELTEVFEFA | 20 | HPV18-E6 | A*02:01 |
| CD8⁺ | TVLELTEVFEF | 21 | HPV18-E6 | A*02:01 |
| CD8⁺ | LLMGTLGIV | 22 | HPV16-E7 | A*02:01 |
| CD8⁺ | DRAHYNIVTF | 23 | HPV16-E7 | B*27:05 |
| CD8⁺ | LEDLLMGTLGI | 24 | HPV16-E7 | C*04:01 |
| CD4⁺ | WKSFFSRTWSRLSLH | 25 | HPV16-E1 | DRB1*04:01 |
| CD4⁺ | HIDYWKHMRLECALY | 26 | HPV16-E2 | DQB1*03:01 |
| CD4⁺ | SVDSAPILTAFNSSH | 27 | HPV16-E2 | DQB1*03:01 |
| CD4⁺ | VYDYAFRDLCIVYRDGNPYAVCD | 28 | HPV16-E6 | DRB1*15:01 |
| CD4⁺ | RCINCQKPLCPEEKQRHLD | 29 | HPV16-E6 | DRB1*15:01 |
| CD4⁺ | EKQRHLDKKQRFHNIRGRWTGRCMSCC | 30 | HPV16-E6 | DRB1*15:01 |
| CD4⁺ | TPTLHEYMLDLQPETTDLY | 31 | HPV16-E7 | DQB1*03:01 |
| CD4⁺ | YEQLNDSSEEEDEID | 32 | HPV16-E7 | DQB1*03:01 |
| CD4⁺ | VQSTHVDIRTLEDLLMGTL | 33 | HPV16-E7 | DQB1*03:01 |

In some implementations, the peptides provided herein are full length HPV proteins. In some implementations, the peptides provided herein comprise less than 100, 90, 80, 70, 60, 50, 40, 30, 25, 20, 15 or 10 contiguous amino acids of the HPV viral protein. In some implementations, the peptides provided herein comprise two or more of the HPV epitopes listed in Table 1. For example, in some implementations, the peptide provided herein comprises two or more of the HPV epitopes listed in table 1 connected by polypeptide linkers. In some implementations, the peptide provided herein comprises 2, 3, 4, 5, 6, 7, 8, 9, 10, 11, 12, 13, 14, 15, 16, 17, 18, 19 or 20 of the epitopes listed in Table 1.

In some implementations, the peptide provided herein consists of an epitope listed in Table 1. In some implementations, the peptide provided herein consists essentially of an epitope listed in Table 1. In some implementations, the peptide provided herein comprise no more than 20, 19, 18, 17, 16, 15, 14, 13, 12, 11, 10, 9, 8, 7, 6, 5, 4, 3, 2 or 1 amino acids in addition to the epitopes listed in Table 1.

In some implementations, the sequence of the peptides comprises an HPV viral protein sequence except for 1 or more (*e.g.,* 1, 2, 3, 4, 5, 6, 7, 8, 9, 10 or more) conservative sequence modifications. As used herein, the term "conservative sequence modifications" is intended to refer to amino acid modifications that do not significantly affect or alter the interaction between a TCR and a peptide containing the amino acid sequence presented on an MHC. Such conservative modifications include amino acid substitutions, additions (*e.g.,* additions of amino acids to the N or C terminus of the peptide) and deletions (*e.g*., deletions of amino acids from the N or C terminus of the peptide). Conservative amino acid substitutions are ones in which the amino acid residue is replaced with an amino acid residue having a similar side chain. Families of amino acid residues having similar side chains have been defined in the art. These families include amino acids with basic side chains (*e.g.,* lysine, arginine, histidine), acidic side chains (*e.g.,* aspartic acid, glutamic acid), uncharged polar side chains (*e.g.*, glycine, asparagine, glutamine, serine, threonine, tyrosine, cysteine, tryptophan), nonpolar side chains (*e.g*., alanine, valine, leucine, isoleucine, proline, phenylalanine, methionine), beta-branched side chains (*e.g*., threonine, valine, isoleucine) and aromatic side chains (*e.g.*, tyrosine, phenylalanine, tryptophan, histidine). Thus, one or more amino acid residues of the peptides described herein can be replaced with other amino acid residues from the same side chain family and the altered peptide can be tested for retention of TCR binding using methods known in the art. Modifications can be introduced into an antibody by standard techniques known in the art, such as site-directed mutagenesis and PCR-mediated mutagenesis.

To determine the percent identity of two amino acid sequences or of two nucleic acid sequences, the sequences are aligned for optimal comparison purposes (*e.g*., gaps can be introduced in one or both of a first and a second amino acid or nucleic acid sequence for optimal alignment and non-identical sequences can be disregarded for comparison purposes). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences, taking into account the number of gaps, and the length of each gap, which need to be introduced for optimal alignment of the two sequences.

Also provided herein are chimeric or fusion proteins. As used herein, a "chimeric protein" or "fusion protein" comprises a peptide(s) provided herein (*e.g*., those comprising an epitope listed in Table 1) linked to a distinct peptide to which it is not linked in nature. For example, the distinct peptide can be fused to the N-terminus or C-terminus of the peptide either directly, through a peptide bond, or indirectly through a chemical linker. In some implementations, the peptide of the provided herein is linked to polypeptides comprising other HPV epitopes. In some implementations, the peptide provided herein is linked to peptides comprising epitopes from other viral and/or infectious diseases. In some implementations, the peptide provided herein is linked to a peptide encoding a cancer-associated epitope.

A chimeric or fusion peptide provided herein can be produced by standard recombinant DNA techniques. For example, DNA fragments coding for the different peptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another implementation, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and re-amplified to generate a chimeric gene sequence (see, for example, Current Protocols in Molecular Biology, Ausubel et al., eds., John Wiley & Sons: 1992). Moreover, many expression vectors are commercially available that already encode a fusion moiety.

In some aspects, provided herein are cells that present a peptide described herein (*e.g.,* a peptide comprising an epitope listed in Table 1). In some implementations, the cell is a mammalian cell. In some implementations the cell is an antigen presenting cell (APC) (*e.g.,* an antigen presenting t-cell, a dendritic cell, a B cell, a macrophage or am artificial antigen presenting cell, such as aK562 cell). A cell presenting a peptide described herein can be produced by standard techniques known in the art. For example, a cell may be pulsed to encourage peptide uptake. In some implementations, the cells are transfected with a nucleic acid encoding a peptide provided herein. In some aspects, provided herein are methods of producing antigen presenting cells (APCs), comprising pulsing a cell with the peptides described herein. Exemplary examples of producing antigen presenting cells can be found in WO2013088114.

The peptides provided herein can be isolated from cells or tissue sources by an appropriate purification scheme using standard protein purification techniques, can be produced by recombinant DNA techniques, and/or can be chemically synthesized using standard peptide synthesis techniques. The peptides described herein can be produced in prokaryotic or eukaryotic host cells by expression of nucleotides encoding a peptide(s) of the present disclosure. Alternatively, such peptides can be synthesized by chemical methods. Methods for expression of heterologous peptides in recombinant hosts, chemical synthesis of peptides, and *in vitro* translation are well known in the art and are described further in Maniatis et al., Molecular Cloning: A Laboratory Manual (1989), 2nd Ed., Cold Spring Harbor, N. Y.; Berger and Kimmel, Methods in Enzymology, Volume 152, Guide to Molecular Cloning Techniques (1987), Academic Press, Inc., San Diego, Calif.; Merrifield, J. (1969) J. Am. Chem. Soc. 91:501; Chaiken I. M. (1981) CRC Crit. Rev. Biochem. 11:255; Kaiser et al. (1989) Science 243:187; Merrifield, B. (1986) Science 232:342; Kent, S. B. H. (1988) Annu. Rev. Biochem. 57:957; and Offord, R. E. (1980) Semisynthetic Proteins, Wiley Publishing.

### Nucleic Acid Molecules

Provided herein are nucleic acid molecules that encode the peptides described herein. In some aspects, provided herein are methods of treating cancer, precancerous lesions, or HPV by administering to a subject the nucleic acids disclosed herein. The nucleic acids may be present, for example, in whole cells, in a cell lysate, or in a partially purified or substantially pure form.

In some implementations, provided herein are vectors (*e.g.,* a viral vector, such as an adenovirus based expression vector) that contain the nucleic acid molecules described herein. As used herein, the term "vector," refers to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (*e.g.*, bacterial vectors having a bacterial origin of replication, episomal mammalian vectors). Other vectors (*e.g*., non-episomal mammalian vectors) can be integrated into the genome of a host cell upon introduction into the host cell, and thereby be replicated along with the host genome. Moreover, certain vectors are capable of directing the expression of genes. Such vectors are referred to herein as "recombinant expression vectors" (or simply, "expression vectors"). In some implementations, provided herein are nucleic acids operable linked to one or more regulatory sequences (*e.g.,* a promoter) in an expression vector. In some implementations the cell transcribes the nucleic acid provided herein and thereby expresses an antibody, antigen binding fragment thereof or peptide described herein. The nucleic acid molecule can be integrated into the genome of the cell or it can be extrachromosomal.

In some implementations, the nucleic acid provided herein is part of a vaccine. In some implementations, the vaccine is delivered to a subject in a vector, including, but not limited to, a bacterial vector and/or a viral vector. Examples of bacterial vectors include, but are not limited to, Mycobacterium bovis (BCG), Salmonella Typhimurium ssp., Salmonella Typhi ssp., Clostridium sp. spores, Escherichia coli Nissle 1917, Escherichia coli K-12/LLO, Listeria monocytogenes, and Shigella flexneri. Examples of viral vectors include, but are not limited to, vaccinia, adenovirus, RNA viruses (replicons), and replication-defective like avipox, fowlpox, canarypox, MVA, and adenovirus.

In some implementations, provided herein are cells that contain a nucleic acid described herein (*e.g.,* a nucleic acid encoding an antibody, antigen binding fragment thereof or peptide described herein). The cell can be, for example, prokaryotic, eukaryotic, mammalian, avian, murine and/or human. In some implementations, the cell is a mammalian cell. In some implementations the cell is an APC (*e.g.* an antigen presenting T cell, a dendritic cell, a B cell, or an aK562 cell). In the present methods, a nucleic acid described herein can be administered to the cell, for example, as nucleic acid without delivery vehicle, in combination with a delivery reagent. In some implementations, any nucleic acid delivery method known in the art can be used in the methods described herein. Suitable delivery reagents include, but are not limited to, *e.g.,* the Mirus Transit TKO lipophilic reagent; lipofectin; lipofectamine; cellfectin; polycations (*e.g.*, polylysine), atelocollagen, nanoplexes and liposomes. In some implementations of the methods described herein, liposomes are used to deliver a nucleic acid to a cell or subject. Liposomes suitable for use in the methods described herein can be formed from standard vesicle-forming lipids, which generally include neutral or negatively charged phospholipids and a sterol, such as cholesterol. The selection of lipids is generally guided by consideration of factors such as the desired liposome size and half-life of the liposomes in the blood stream. A variety of methods are known for preparing liposomes, for example, as described in Szoka et al. (1980), Ann. Rev. Biophys. Bioeng. 9:467; and U.S. Pat. Nos. 4,235,871, 4,501,728, 4,837,028, and 5,019,369.

### Antibodies

In some aspects, the compositions and methods provided herein relate to antibodies and antigen-binding fragments thereof that bind specifically to a protein expressed on the plasma membrane of an HPV infected cell, or a cancer cell (*e.g.,* a protein comprising the epitope listed in Table 1), or precancerous lesions. In some implementations, the antibodies bind to a particular epitope of one of the peptides provided herein. In some implementations, an antibody that binds to an HPV protein comprising an epitope with an amino acid sequence in Table 1, wherein the HPV protein is not a full length HPV protein. In some implementations, the epitope is an extracellular epitope. In some implementations, the epitope is an epitope listed in Table 1. In some implementation , the HPV epitopes are from HPV E1, E2, E4, E5, E6, and/or E7. In some implementations, the HPV is type 16 or 18. In some implementations, the antibodies can be polyclonal or monoclonal and can be, for example, murine, chimeric, humanized or fully human. In some implementations, the antibody is a full-length immunoglobulin molecule, an scFv, a Fab fragment, an Fab' fragment, a F(ab')2 fragment, an Fv, a camelid antibody a disulfide linked Fv or a Designed Ankyrin Repeat Protein (DARPin).

Polyclonal antibodies can be prepared by immunizing a suitable subject (*e.g*. a mouse) with a peptide immunogen (*e.g.*, an amino acid sequence listed in Table 1). In some implementations, the peptide immunogen comprises an extracellular epitope of a target protein provided herein. The peptide antibody titer in the immunized subject can be monitored over time by standard techniques, such as with an enzyme linked immunosorbent assay (ELISA) using immobilized peptide. If desired, the antibody directed against the antigen can be isolated from the mammal (*e.g*., from the blood) and further purified by well-known techniques, such as protein A chromatography to obtain the IgG fraction.

At an appropriate time after immunization, *e.g*., when the antibody titers are highest, antibody-producing cells can be obtained from the subject and used to prepare monoclonal antibodies using standard techniques, such as the hybridoma technique originally described by Kohler and Milstein (1975) Nature 256:495-497) (see also Brown et al. (1981) J. Immunol. 127:539-46; Brown et al. (1980) J. Biol. Chem. 255:4980-83; Yeh et al. (1976) Proc. Natl. Acad. Sci. 76:2927-31; and Yeh et al. (1982) Int. J. Cancer 29:269-75), a human B cell hybridoma technique (Kozbor et al. (1983) Immunol. Today 4:72), an EBV-hybridoma technique (Cole et al. (1985) Monoclonal Antibodies and Cancer Therapy, Alan R. Liss, Inc., pp. 77-96) or a trioma techniques. The technology for producing monoclonal antibody hybridomas is well known (see generally Kenneth, R. H. in Monoclonal Antibodies: A New Dimension In Biological Analyses, Plenum Publishing Corp., New York, New York (1980); Lerner, E. A. (1981) Yale J. Biol. Med. 54:387-402; Gefter, M. L. et al. (1977) Somatic Cell Genet. 3:231-36). Briefly, an immortal cell line (typically a myeloma) is fused to lymphocytes (typically splenocytes) from a mammal immunized with an immunogen as described above, and the culture supernatants of the resulting hybridoma cells are screened to identify a hybridoma producing a monoclonal antibody that binds to the peptide antigen, preferably specifically.

As an alternative to preparing monoclonal antibody-secreting hybridomas, a monoclonal antibody that binds to a target protein described herein can be obtained by screening a recombinant combinatorial immunoglobulin library with the appropriate peptide (*e.g.* a peptide comprising an epitope of Table 1) to thereby isolate immunoglobulin library members that bind the peptide.

Additionally, recombinant antibodies specific for a target protein provided herein and/or an extracellular epitope of a target protein provided herein, such as chimeric or humanized monoclonal antibodies, can be made using standard recombinant DNA techniques. Such chimeric and humanized monoclonal antibodies can be produced by recombinant DNA techniques known in the art, for example using methods described in US Pat No. 4,816,567; US Pat. No. 5,565,332; Better et al. (1988) Science 240:1041-1043; Liu et al. (1987) Proc. Natl. Acad. Sci. USA 84:3439-3443; Liu et al. (1987) J. Immunol. 139:3521-3526; Sun et al. (1987) Proc. Natl. Acad. Sci. 84:214-218; Nishimura et al. (1987) Cancer Res. 47:999-1005; Wood et al. (1985) Nature 314:446-449; and Shaw et al. (1988) J. Natl. Cancer Inst. 80:1553-1559); Morrison, S. L. (1985) Science 229:1202-1207; Oi et al. (1986) Biotechniques 4:214; Winter U.S. Patent 5,225,539; Jones et al. (1986) Nature 321:552-525; Verhoeyan et al. (1988) Science 239:1534; and Beidler et al. (1988) J. Immunol. 141:4053-4060.

Human monoclonal antibodies specific for a target protein provided herein and/or an extracellular epitope provided herein can be generated using transgenic or transchromosomal mice carrying parts of the human immune system rather than the mouse system. For example, "HuMAb mice" which contain a human immunoglobulin gene miniloci that encodes unrearranged human heavy (µ and γ) and κ light chain immunoglobulin sequences, together with targeted mutations that inactivate the endogenous µ and κ chain loci (Lonberg, N. et al. (1994) Nature 368(6474): 856 859). Accordingly, the mice exhibit reduced expression of mouse IgM or κ, and in response to immunization, the introduced human heavy and light chain transgenes undergo class switching and somatic mutation to generate high affinity human IgGκ monoclonal antibodies (Lonberg, N. *et al.* (1994), supra; reviewed in Lonberg, N. (1994) Handbook of Experimental Pharmacology 113:49 101; Lonberg, N. and Huszar, D. (1995) Intern. Rev. Immunol. Vol. 13: 65 93, and Harding, F. and Lonberg, N. (1995) Ann. N. Y Acad. Sci 764:536 546). The preparation of HuMAb mice is described in Taylor, L. et al. (1992) Nucleic Acids Research 20:6287 6295; Chen, J. et al. (1993) International Immunology 5: 647 656; Tuaillon et al. (1993) Proc. Natl. Acad. Sci USA 90:3720 3724; Choi et al. (1993) Nature Genetics 4:117 123; Chen, J. et al. (1993) EMBO J. 12: 821 830; Tuaillon et al. (1994) J. Immunol. 152:2912 2920; Lonberg et al., (1994) Nature 368(6474): 856 859; Lonberg, N. (1994) Handbook of Experimental Pharmacology 113:49 101; Taylor, L. et al. (1994) International Immunology 6: 579 591; Lonberg, N. and Huszar, D. (1995) Intern. Rev. Immunol. Vol. 13: 65 93; Harding, F. and Lonberg, N. (1995) Ann. N.Y. Acad. Sci 764:536 546; Fishwild, D. et al. (1996) Nature Biotechnology 14: 845 851. See further, U.S. Pat. Nos. 5,545,806; 5,569,825; 5,625,126; 5,633,425; 5,789,650; 5,877,397; 5,661,016; 5,814,318; 5,874,299; 5,770,429; and 5,545,807.

In some implementations, the antibodies provided herein are able to bind to an epitope listed in Tables 1 with a dissociation constant of no greater than 10⁻⁶, 10⁻⁷, 10⁻⁸ or 10⁻⁹ M. Standard assays to evaluate the binding ability of the antibodies are known in the art, including for example, ELISAs, Western blots and RIAs. The binding kinetics (e.g., binding affinity) of the antibodies also can be assessed by standard assays known in the art, such as by Biacore analysis.

In some implementations the antibody is part of an antibody-drug conjugate. Antibody-drug conjugates are therapeutic molecules comprising an antibody (*e.g*., an antibody that binds to a protein listed in Table 1) linked to a biologically active agent, such as a cytotoxic agent or an antiviral agent. In some implementations, the biologically active agent is linked to the antibody via a chemical linker. Such linkers can be based on any stable chemical motif, including disulfides, hydrazones, peptides or thioethers. In some implementations, the linker is a cleavable linker and the biologically active agent is released from the antibody upon antibody binding to the plasma membrane target protein. In some implementations, the linker is a noncleavable linker.

In some implementations, the antibody-drug conjugate comprises an antibody linked to a cytotoxic agent. In some implementations, any cytotoxic agent able to kill HPV infected cells can be used. In some implementations, the cytotoxic agent is MMAE, DM-1, a maytansinoid, a doxorubicin derivative, an auristatin, a calcheamicin, CC-1065, an aduocarmycin or an anthracycline.

In some implementations, the antibody-drug conjugate comprises an antibody linked to an antiviral agent. In some implementations, any antiviral agent capable of inhibiting HPV replication is used. In some implementations, the antiviral agent is ganciclovir, valganciclovir, foscarnet, cidofovir, acyclovir, formivirsen, maribavir, BAY 38-4766 or GW275175X. In some implementations, provided herein are vaccines composing the antibodies or antibody-drug conjugates described herein.

### Cells

In some aspects, provided herein are antigen presenting cells (APCs) that express on their surface a MHC that present one or more peptides comprising an HPV epitope described herein (*e.g.,* APCs that present one or more of the HPV epitopes listed in Table 1). In some implementations, the MHC is a class I MHC. In some implementations, the MHC is a class II MHC. In some implementations, the class I MHC has an α chain polypeptide that is HLA-A, HLA-B, HLA-C, HLA-E, HLA-F, HLA-g, HLA-K or HLA-L. In some implementation, the class II MHC has an α chain polypeptide that is HLA-DMA, HLA-DOA, HLA-DPA, HLA-DQA or HLA-DRA. In some implementations, the class II MHC has a β chain polypeptide that is HLA-DMB, HLA-DOB, HLA-DPB, HLA-DQB or HLA-DRB.

In some implementations the APCs are B cells, antigen presenting T-cells, dendritic cells, or artificial antigen-presenting cells (*e.g.,* aK562 cells). Dendritic cells for use in the process may be prepared by taking PBMCs from a patient sample and adhering them to plastic. Generally, the monocyte population sticks and all other cells can be washed off. The adherent population is then differentiated with IL-4 and GM-CSF to produce monocyte derived dendritic cells. These cells may be matured by the addition of IL-1β, IL-6, PGE-1 and TNF-α (which upregulates the important co-stimulatory molecules on the surface of the dendritic cell) and are then transduced with one or more of the peptides provided herein.

In some implementations, the APC is an artificial antigen-presenting cell, such as an aK562 cell. In some implementations, the artificial antigen-presenting cells are engineered to express CD80, CD83, 41BB-L, and/or CD86. Exemplary artificial antigen-presenting cells, including aK562 cells, are described U.S. Pat. Pub. No. 2003/0147869.

In certain aspects, provided herein are methods of generating APCs that present the one or more of the HPV epitopes described herein comprising contacting an APC with a peptide comprising an HPV epitope described herein and/or with a nucleic acid encoding an HPV epitope described herein. In some implementations, the APCs are irradiated.

In certain aspects, provided herein are T cells (*e.g.,* CD4 T cells and/or CD8 T cells) that express a TCR (*e.g.,* an αβ TCR or a γδ TCR) that recognizes a peptide described herein (a peptide comprising an HPV epitope listed in Table 1) presented on a MHC. In some implementations, the T cell is a CD8 T cell (a CTL) that expresses a TCR that recognizes a peptide described herein presented on a class I MHC. In some implementations, the T cell is a CD4 T cell (a helper T cell) that recognizes a peptide described herein presented on a class II MHC.

In some aspects, provided herein are methods of generating, activating and/or inducing proliferation of T cells (*e.g.,* CTLs) that recognize one or more of the HPV epitopes described herein. In some implementations, a sample comprising CTLs (*i.e.,* a PBMC sample) is incubated in culture with an APC provided herein (*e.g.,* an APCs that present a peptide comprising an HPV epitope described herein on a class I MHC complex). In some implementations, the APCs are autologous to the subject from whom the T cells were obtained. In some implementations, the sample containing T cells are incubated 2 or more times with APCs provided herein. In some implementations, the T cells are incubated with the APCs in the presence of at least one cytokine. In some implementations, the cytokine is IL-4, IL-7 and/or IL-15. Exemplary methods for inducing proliferation of T cells using APCs are provided, for example, in U.S. Pat. Pub. No. 2015/0017723.

In some aspects, provided herein are compositions (*e.g.*, prophylactic and/or therapeutic compositions) comprising T cells and/or APCs provided herein. In some implementations, such compositions are used to treat and/or prevent a cancer, and/or precancerous lesions and/or an HPV infection in a subject by administering to the subject an effective amount of the composition In some implementations, the T cells and/or APCs are not autologous to the subject. In some implementations, the T cells and/or APCs are autologous to the subject. In some implementations, the T cells and/or APCs are stored in a cell bank before they are administered to the subject.

### Pharmaceutical Compositions

In some aspects, provided herein is a composition (*e.g.*, a pharmaceutical composition, such as a vaccine composition), containing a peptide (*e.g*., comprising an epitope from Table 1), nucleic acid, antibody, CTL, or an APC described herein formulated together with a pharmaceutically acceptable carrier, as well as methods of treating cancer, precancerous lesions, or an HPV infection using such pharmaceutical compositions. In some implementations, the composition includes a combination of multiple (*e.g.*, two or more) agents provided herein.

In some implementations, the pharmaceutical composition further comprises an adjuvant. As used herein, the term *"adjuvant"* broadly refers to an agent that affects an immunological or physiological response in a patient or subject. For example, an adjuvant might increase the presence of an antigen over time or to an area of interest like a tumor, help absorb an antigen-presenting cell antigen, activate macrophages and lymphocytes and support the production of cytokines. By changing an immune response, an adjuvant might permit a smaller dose of an immune interacting agent to increase the effectiveness or safety of a particular dose of the immune interacting agent. For example, an adjuvant might prevent T cell exhaustion and thus increase the effectiveness or safety of a particular immune interacting agent. Examples of adjuvants include, but are not limited to, an immune modulatory protein, Adjuvant 65, α-GalCer, aluminum phosphate, aluminum hydroxide, calcium phosphate, β-Glucan Peptide, CpG DNA, GPI-0100, lipid A, lipopolysaccharide, Lipovant, Montanide, N-acetyl-muramyl-L-alanyl-D-isoglutamine, Pam3CSK4, quil A and trehalose dimycolate.

Methods of preparing these formulations or compositions include the step of bringing into association an agent described herein with the carrier and, optionally, one or more accessory ingredients. In general, the formulations are prepared by uniformly and intimately bringing into association an agent described herein with liquid carriers, or finely divided solid carriers, or both, and then, if necessary, shaping the product.

Pharmaceutical compositions of this disclosure suitable for parenteral administration comprise one or more agents described herein in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain sugars, alcohols, antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the disclosure include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

Regardless of the route of administration selected, the agents of the present disclosure, which may be used in a suitable hydrated form, and/or the pharmaceutical compositions of the present invention, are formulated into pharmaceutically-acceptable dosage forms by conventional methods known to those of skill in the art.

### Therapeutic Methods

New treatment approaches take advantage of our knowledge of how the immune system can eliminate virus-infected cells, which is done mainly by cytotoxic T cells (also known as "killer" T cells). These new therapies involve the use of anti-cancer vaccines and intra-lesion immune system-based therapy, with the idea to activate T cells so they can then locate and kill HPV-infected cells.

In certain implementations, provided herein are methods of treating an HPV infection, and/or a cancer, and/or precancerous lesions in a subject comprising administering to the subject a pharmaceutical composition provided herein.

In some implementations, provided herein is a method of treating an HPV infection in a subject. In some implementations, the subject treated is immunocompromised. For example, in some implementations, the subject has a T cell deficiency. In some implementations, the subject has leukemia, lymphoma or multiple myeloma. In some implementations, the subject is infected with HIV and/or has AIDS. In some implementations, the subject has undergone a tissue, organ and/or bone marrow transplant. In some implementations, the subject is being administered immunosuppressive drugs. In some implementations, the subject has undergone and/or is undergoing a chemotherapy. In some implementations, the subject has undergone and/or is undergoing radiation therapy.

In some implementations, the subject is also administered an anti-viral drug that inhibits HPV replication. For example, in some implementations, the subject is administered podofilox, imiquimod, sinecatechins, podophyllin resin, trichloroacetic acid, or bichloracetic acid. In some implementations, the subject is also treated with an intervention that physically affects the HPV infected lesions and/or HPV-associated tumors. For example, in some implementations, the lesions are treated with surgical excision, chemical ablation, cryotherapy, or cauterization.

In some implementations, the subject has cancer or precancerous lesions. In some implementations, the methods described herein may be used to treat any cancerous or pre-cancerous tumor. In some implementations, the cancer and/or precancerous lesions expresse one or more of the HPV epitopes provided herein (*e.g.,* the HPV epitopes listed in Table 1). In some implementations, the precancerous lesions include abnormal cell changes and/or precancerous cell changes. Precancerous lesions that may be treated by methods and compositions provided herein include, but are not limited to, cervical intraepithelial neoplasia (CIN), squamous intraepithelial lesions (SIL), or warts on the cervix. In some implementations, the cancer includes a solid tumor. Cancers that may be treated by methods and compositions provided herein include, but are not limited to, cancer cells from the cervix, anus, vagina, vulva, penis, tongue base, larynx, tonsil, bladder, blood, bone, bone marrow, brain, breast, colon, esophagus, gastrointestine, gum, head, kidney, liver, lung, nasopharynx, neck, ovary, prostate, skin, non-melanoma skin cancer (NMSC), cutaneous squamous cell carcinoma (SCC), stomach, testis, tongue, or uterus. In addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometrioid carcinoma; skin appendage carcinoma; apocrine adenocarcinoma; sebaceous adenocarcinoma; ceruminous adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; mammary paget's disease; acinar cell carcinoma; adenosquamous carcinoma; adenocarcinoma w/squamous metaplasia; malignant thymoma; malignant ovarian stromal tumor; malignant thecoma; malignant granulosa cell tumor; and malignant roblastoma; sertoli cell carcinoma; malignant leydig cell tumor; malignant lipid cell tumor; malignant paraganglioma; malignant extra-mammary paraganglioma; pheochromocytoma; glomangiosarcoma; malignant melanoma; amelanotic melanoma; superficial spreading melanoma; malignant melanoma in giant pigmented nevus; epithelioid cell melanoma; malignant blue nevus; sarcoma; fibrosarcoma; malignant fibrous histiocytoma; myxosarcoma; liposarcoma; leiomyosarcoma; rhabdomyosarcoma; embryonal rhabdomyosarcoma; alveolar rhabdomyosarcoma; stromal sarcoma; malignant mixed tumor; mullerian mixed tumor; nephroblastoma; hepatoblastoma; carcinosarcoma; malignant mesenchymoma; malignant brenner tumor; malignant phyllodes tumor; synovial sarcoma; malignant mesothelioma; dysgerminoma; embryonal carcinoma; malignant teratoma; malignant struma ovarii; choriocarcinoma; malignant mesonephroma; hemangiosarcoma; malignant hemangioendothelioma; kaposi's sarcoma; malignant hemangiopericytoma; lymphangiosarcoma; osteosarcoma; juxtacortical osteosarcoma; chondrosarcoma; malignant chondroblastoma; mesenchymal chondrosarcoma; giant cell tumor of bone; ewing's sarcoma; malignant odontogenic tumor; ameloblastic odontosarcoma; malignant ameloblastoma; ameloblastic fibrosarcoma; malignant pinealoma; chordoma; malignant glioma; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; malignant meningioma; neurofibrosarcoma; malignant neurilemmoma; malignant granular cell tumor; malignant lymphoma; Hodgkin's disease; Hodgkin's lymphoma; paragranuloma; small lymphocytic malignant lymphoma; diffuse large cell malignant lymphoma; follicular malignant lymphoma; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia.

In some implementations, the subject is also administered an anti-cancer compound. Exemplary anti-cancer compounds include, but are not limited to, Alemtuzumab (Campath^{®}), Alitretinoin (Panretin^{®}), Anastrozole (Arimidex^{®}), Bevacizumab (Avastin^{®}), Bexarotene (Targretin^{®}), Bortezomib (Velcade^{®}), Bosutinib (Bosulif^{®}), Brentuximab vedotin (Adcetris^{®}), Cabozantinib (Cometriq^{™}), Carfilzomib (Kyprolis^{™}), Cetuximab (Erbitux^{®}), Crizotinib (Xalkori^{®}), Dasatinib (Sprycel^{®}), Denileukin diftitox (Ontak^{®}), Erlotinib hydrochloride (Tarceva^{®}), Everolimus (Afinitor^{®}), Exemestane (Aromasin^{®}), Fulvestrant (Faslodex^{®}), Gefitinib (Iressa^{®}), Ibritumomab tiuxetan (Zevalin^{®}), Imatinib mesylate (Gleevec^{®}), Ipilimumab (Yervoy^{™}), Lapatinib ditosylate (Tykerb^{®}), Letrozole (Femara^{®}), Nilotinib (Tasigna^{®}), Ofatumumab (Arzerra^{®}), Panitumumab (Vectibix^{®}), Pazopanib hydrochloride (Votrient^{®}), Pertuzumab (Perjeta^{™}), Pralatrexate (Folotyn^{®}), Regorafenib (Stivarga^{®}), Rituximab (Rituxan^{®}), Romidepsin (Istodax^{®}), Sorafenib tosylate (Nexavar^{®}), Sunitinib malate (Sutent^{®}), Tamoxifen, Temsirolimus (Torisel^{®}), Toremifene (Fareston^{®}), Tositumomab and 131I-tositumomab (Bexxar^{®}), Trastuzumab (Herceptin^{®}), Tretinoin (Vesanoid^{®}), Vandetanib (Caprelsa^{®}), Vemurafenib (Zelboraf^{®}), Vorinostat (Zolinza^{®}), and Ziv-aflibercept (Zaltrap^{®}).

In some implementations, the subject is also administered a chemotherapeutic agent. Examples of such chemotherapeutic agents include, but are not limited to, alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin gammalI and calicheamicin omegal1; dynemicin, including dynemicin A; bisphosphonates, such as clodronate; an esperamicin; as well as neocarzinostatin chromophore and related chromoprotein enediyne antibiotic chromophores, aclacinomysins, actinomycin, authrarnycin, azaserine, bleomycins, cactinomycin, carabicin, caminomycin, carzinophilin, chromomycinis, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK polysaccharide complex); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., paclitaxel and doxetaxel; chlorambucil; gemcitabine; 6-thioguanine; mercaptopurine; methotrexate; platinum coordination complexes such as cisplatin, oxaliplatin and carboplatin; vinblastine; platinum; etoposide (VP-16); ifosfamide; mitoxantrone; vincristine; vinorelbine; novantrone; teniposide; edatrexate; daunomycin; aminopterin; xeloda; ibandronate; irinotecan (e.g., CPT-11); topoisomerase inhibitor RFS 2000; difluoromethylornithine (DMFO); retinoids such as retinoic acid; capecitabine; and pharmaceutically acceptable salts, acids or derivatives of any of the above.

In some implementations, the subject is also administered an immunotherapeutic agent. Immunotherapy refers to a treatment that uses a subject's immune system to treat cancer, *e.g*. cancer vaccines, cytokines, use of cancer-specific antibodies, T cell therapy, and dendritic cell therapy.

In some implementations, the subject is also administered an immune modulatory protein. Examples of immune modulatory proteins include, but are not limited to, B lymphocyte chemoattractant ("BLC"), C-C motif chemokine 11 ("Eotaxin-1"), Eosinophil chemotactic protein 2 ("Eotaxin-2"), Granulocyte colony-stimulating factor ("G-CSF"), Granulocyte macrophage colony-stimulating factor ("GM-CSF"), 1-309, Intercellular Adhesion Molecule 1 ("ICAM-1"), Interferon gamma ("IFN-gamma"), Interlukin-1 alpha ("IL-1 alpha"), Interleukin-1 beta ("IL-1 beta"), Interleukin 1 receptor antagonist ("IL-1 ra"), Interleukin-2 ("IL-2"), Interleukin-4 ("IL-4"), Interleukin-5 ("IL-5"), Interleukin-6 ("IL-6"), Interleukin-6 soluble receptor ("IL-6 sR"), Interleukin-7 ("IL-7"), Interleukin-8 ("IL-8"), Interleukin-10 ("IL-10"), Interleukin- 11 ("IL-11"), Subunit beta of Interleukin-12 ("IL-12 p40" or "IL-12 p70"), Interleukin-13 ("IL-13"), Interleukin-15 ("IL-15"), Interleukin-16 ("IL-16"), Interleukin-17 ("IL-17"), Chemokine (C-C motif) Ligand 2 ("MCP-1"), Macrophage colony-stimulating factor ("M-CSF"), Monokine induced by gamma interferon ("MIG"), Chemokine (C-C motif) ligand 2 ("MIP-1 alpha"), Chemokine (C-C motif) ligand 4 ("MIP-1 beta"), Macrophase inflammatory protein-1-delta ("MIP-1 delta"), Platelet-derived growth factor subunit B ("PDGF-BB"), Chemokine (C-C motif) ligand 5, Regulated on Activation, Normal T cell Expressed and Secreted ("RANTES"), TIMP metallopeptidase inhibitor 1 ("TIMP-1"), TIMP metallopeptidase inhibitor 2 ("TIMP-2"), Tumor necrosis factor, lymphotoxin-alpha ("TNF alpha"), Tumor necrosis factor, lymphotoxin-beta ("TNF beta"), Soluble TNF receptor type 1 ("sTNFRI"), sTNFRIIAR, Brain-derived neurotrophic factor ("BDNF"), Basic fibroblast growth factor ("bFGF"), Bone morphogenetic protein 4 ("BMP-4"), Bone morphogenetic protein 5 ("BMP-5"), Bone morphogenetic protein 7 ("BMP-7"), Nerve growth factor ("b-NGF"), Epidermal growth factor ("EGF"), Epidermal growth factor receptor ("EGFR"), Endocrine-gland-derived vascular endothelial growth factor ("EG-VEGF"), Fibroblast growth factor 4 ("FGF-4"), Keratinocyte growth factor ("FGF-7"), Growth differentiation factor 15 ("GDF-15"), Glial cell-derived neurotrophic factor ("GDNF"), Growth Hormone, Heparin-binding EGF-like growth factor ("HB-EGF"), Hepatocyte growth factor ("HGF"), Insulin-like growth factor binding protein 1 ("IGFBP-1"), Insulin-like growth factor binding protein 2 ("IGFBP-2"), Insulin-like growth factor binding protein 3 (" IGFBP-3 "), Insulin-like growth factor binding protein 4 ("IGFBP-4"), Insulin-like growth factor binding protein 6 ("IGFBP-6"), Insulin-like growth factor 1 ("IGF-1"), Insulin, Macrophage colony-stimulating factor ("M-CSF R"), Nerve growth factor receptor ("NGF R"), Neurotrophin-3 ("NT-3"), Neurotrophin-4 ("NT-4"), Osteoclastogenesis inhibitory factor ("Osteoprotegerin"), Platelet-derived growth factor receptors ("PDGF-AA"), Phosphatidylinositol-glycan biosynthesis ("PIGF"), Skp, Cullin, F-box containing complex ("SCF"), Stem cell factor receptor ("SCF R"), Transforming growth factor alpha ("TGFalpha"), Transforming growth factor beta-1 ("TGF beta 1"), Transforming growth factor beta-3 ("TGF beta 3"), Vascular endothelial growth factor ("VEGF"), Vascular endothelial growth factor receptor 2 ("VEGFR2"), Vascular endothelial growth factor receptor 3 ("VEGFR3"), VEGF-D 6Ckine, Tyrosine-protein kinase receptor UFO ("Axl"), Betacellulin ("BTC"), Mucosae-associated epithelial chemokine ("CCL28"), Chemokine (C-C motif) ligand 27 ("CTACK"), Chemokine (C-X-C motif) ligand 16 ("CXCL16"), C-X-C motif chemokine 5 ("ENA-78"), Chemokine (C-C motif) ligand 26 ("Eotaxin-3"), Granulocyte chemotactic protein 2 ("GCP-2"), GRO, Chemokine (C-C motif) ligand 14 ("HCC-l"), Chemokine (C-C motif) ligand 16 ("HCC-4"), Interleukin-9 ("IL-9"), Interleukin-17 F ("IL-17F"), Interleukin-18-binding protein ("IL-18 BPa"), Interleukin-28 A ("IL-28A"), Interleukin 29 ("IL-29"), Interleukin 31 ("IL-31"), C-X-C motif chemokine 10 ("IP-10"), Chemokine receptor CXCR3 ("I-TAC"), Leukemia inhibitory factor ("LIF"), Light, Chemokine (C motif) ligand ("Lymphotactin"), Monocyte chemoattractant protein 2 ("MCP-2"), Monocyte chemoattractant protein 3 ("MCP-3"), Monocyte chemoattractant protein 4 ("MCP-4"), Macrophage-derived chemokine ("MDC"), Macrophage migration inhibitory factor ("MIF"), Chemokine (C-C motif) ligand 20 ("MIP-3 alpha"), C-C motif chemokine 19 ("MIP-3 beta"), Chemokine (C-C motif) ligand 23 ("MPIF-1"), Macrophage stimulating protein alpha chain ("MSPalpha"), Nucleosome assembly protein 1-like 4 ("NAP-2"), Secreted phosphoprotein 1 ("Osteopontin"), Pulmonary and activation-regulated cytokine ("PARC"), Platelet factor 4 ("PF4"), Stroma cell-derived factor- 1 alpha ("SDF-1 alpha"), Chemokine (C-C motif) ligand 17 ("TARC"), Thymus-expressed chemokine ("TECK"), Thymic stromal lymphopoietin ("TSLP 4- IBB"), CD 166 antigen ("ALCAM"), Cluster of Differentiation 80 ("B7-1"), Tumor necrosis factor receptor superfamily member 17 ("BCMA"), Cluster of Differentiation 14 ("CD14"), Cluster of Differentiation 30 ("CD30"), Cluster of Differentiation 40 ("CD40 Ligand"), Carcinoembryonic antigen-related cell adhesion molecule 1 (biliary glycoprotein) ("CEACAM-1"), Death Receptor 6 ("DR6"), Deoxythymidine kinase ("Dtk"), Type 1 membrane glycoprotein ("Endoglin"), Receptor tyrosine-protein kinase erbB-3 ("ErbB3"), Endothelial-leukocyte adhesion molecule 1 ("E-Selectin"), Apoptosis antigen 1 ("Fas"), Fms-like tyrosine kinase 3 ("Flt-3L"), Tumor necrosis factor receptor superfamily member 1 ("GITR"), Tumor necrosis factor receptor superfamily member 14 ("HVEM"), Intercellular adhesion molecule 3 ("ICAM-3"), IL-1 R4, IL-1 RI, IL-10 Rbeta, IL-17R, IL-2Rgamma, IL-21R, Lysosome membrane protein 2 ("LIMPII"), Neutrophil gelatinase-associated lipocalin ("Lipocalin-2"), CD62L ("L-Selectin"), Lymphatic endothelium ("LYVE-1"), MHC class I polypeptide-related sequence A ("MICA"), MHC class I polypeptide-related sequence B ("MICB"), NRGl-betal, Beta-type platelet-derived growth factor receptor ("PDGF Rbeta"), Platelet endothelial cell adhesion molecule ("PECAM-1"), RAGE, Hepatitis A virus cellular receptor 1 ("TIM-1"), Tumor necrosis factor receptor superfamily member IOC ("TRAIL R3"), Trappin protein transglutaminase binding domain ("Trappin-2"), Urokinase receptor ("uPAR"), Vascular cell adhesion protein 1 ("VCAM-1"), XEDAR, Activin A, Agouti-related protein ("AgRP"), Ribonuclease 5 ("Angiogenin"), Angiopoietin 1, Angiostatin, Cathepsin S, CD40, Cryptic family protein IB ("Cripto-1"), DAN, Dickkopf-related protein 1 ("DKK-1"), E-Cadherin, Epithelial cell adhesion molecule ("EpCAM"), Fas Ligand (FasL or CD95L), Fcg RIIB/C, FoUistatin, Galectin-7, Intercellular adhesion molecule 2 ("ICAM-2"), IL-13 Rl, IL-13R2, IL-17B, IL-2 Ra, IL-2 Rb, IL-23, LAP, Neuronal cell adhesion molecule ("NrCAM"), Plasminogen activator inhibitor- 1 ("PAI-1"), Platelet derived growth factor receptors ("PDGF-AB"), Resistin, stromal cell-derived factor 1 ("SDF-1 beta"), sgpl30, Secreted frizzled-related protein 2 ("ShhN"), Sialic acid-binding immunoglobulin-type lectins ("Siglec-5"), ST2, Transforming growth factor-beta 2 ("TGF beta 2"), Tie-2, Thrombopoietin ("TPO"), Tumor necrosis factor receptor superfamily member 10D ("TRAIL R4"), Triggering receptor expressed on myeloid cells 1 ("TREM-1"), Vascular endothelial growth factor C ("VEGF-C"), VEGFRl, Adiponectin, Adipsin ("AND"), Alpha-fetoprotein ("AFP"), Angiopoietin-like 4 ("ANGPTL4"), Beta-2-microglobulin ("B2M"), Basal cell adhesion molecule ("BCAM"), Carbohydrate antigen 125 ("CA125"), Cancer Antigen 15-3 ("CA15-3"), Carcinoembryonic antigen ("CEA"), cAMP receptor protein ("CRP"), Human Epidermal Growth Factor Receptor 2 ("ErbB2"), Follistatin, Follicle-stimulating hormone ("FSH"), Chemokine (C-X-C motif) ligand 1 ("GRO alpha"), human chorionic gonadotropin ("beta HCG"), Insulin-like growth factor 1 receptor ("IGF-1 sR"), IL-1 sRII, IL-3, IL-18 Rb, IL-21, Leptin, Matrix metalloproteinase-1 ("MMP-1"), Matrix metalloproteinase-2 ("MMP-2"), Matrix metalloproteinase-3 ("MMP-3"), Matrix metalloproteinase-8 ("MMP-8"), Matrix metalloproteinase-9 ("MMP-9"), Matrix metalloproteinase-10 ("MMP-10"), Matrix metalloproteinase-13 ("MMP-13"), Neural Cell Adhesion Molecule ("NCAM-1"), Entactin ("Nidogen-1"), Neuron specific enolase ("NSE"), Oncostatin M ("OSM"), Procalcitonin, Prolactin, Prostate specific antigen ("PSA"), Sialic acid-binding Ig-like lectin 9 ("Siglec-9"), ADAM 17 endopeptidase ("TACE"), Thyroglobulin, Metalloproteinase inhibitor 4 ("TIMP-4"), TSH2B4, Disintegrin and metalloproteinase domain-containing protein 9 ("ADAM-9"), Angiopoietin 2, Tumor necrosis factor ligand superfamily member 13/ Acidic leucine-rich nuclear phosphoprotein 32 family member B ("APRIL"), Bone morphogenetic protein 2 ("BMP-2"), Bone morphogenetic protein 9 ("BMP-9"), Complement component 5a ("C5a"), Cathepsin L, CD200, CD97, Chemerin, Tumor necrosis factor receptor superfamily member 6B ("DcR3"), Fatty acid-binding protein 2 ("FABP2"), Fibroblast activation protein, alpha ("FAP"), Fibroblast growth factor 19 ("FGF-19"), Galectin-3, Hepatocyte growth factor receptor ("HGF R"), IFN-alpha/beta R2, Insulin-like growth factor 2 ("IGF-2"), Insulin-like growth factor 2 receptor ("IGF-2 R"), Interleukin-1 receptor 6 ("IL-1R6"), Interleukin 24 ("IL-24"), Interleukin 33 ("IL-33", Kallikrein 14, Asparaginyl endopeptidase ("Legumain"), Oxidized low-density lipoprotein receptor 1 ("LOX-1"), Mannose-binding lectin ("MBL"), Neprilysin ("NEP"), Notch homolog 1, translocation-associated (Drosophila) ("Notch-1"), Nephroblastoma overexpressed ("NOV"), Osteoactivin, Programmed cell death protein 1 ("PD-1"), N-acetylmuramoyl-L-alanine amidase ("PGRP-5"), Serpin A4, Secreted frizzled related protein 3 ("sFRP-3"), Thrombomodulin, Toll-like receptor 2 ("TLR2"), Tumor necrosis factor receptor superfamily member 10A ("TRAIL Rl"), Transferrin ("TRF"), WIF-1ACE-2, Albumin, AMICA, Angiopoietin 4, B-cell activating factor ("BAFF"), Carbohydrate antigen 19-9 ("CA19-9"), CD 163 , Clusterin, CRT AM, Chemokine (C-X-C motif) ligand 14 ("CXCL14"), Cystatin C, Decorin ("DCN"), Dickkopf-related protein 3 ("Dkk-3 "), Delta-like protein 1 ("DLL1 "), Fetuin A, Heparin-binding growth factor 1 ("aFGF"), Folate receptor alpha ("FOLR1"), Furin, GPCR-associated sorting protein 1 ("GASP-1"), GPCR-associated sorting protein 2 ("GASP-2"), Granulocyte colony-stimulating factor receptor ("GCSF R"), Serine protease hepsin ("HAI-2"), Interleukin-17B Receptor ("IL-17B R"), Interleukin 27 ("IL-27"), Lymphocyte-activation gene 3 ("LAG-3"), Apolipoprotein A-V ("LDL R"), Pepsinogen I, Retinol binding protein 4 ("RBP4"), SOST, Heparan sulfate proteoglycan ("Syndecan-1"), Tumor necrosis factor receptor superfamily member 13B ("TACI"), Tissue factor pathway inhibitor ("TFPI"), TSP-1, Tumor necrosis factor receptor superfamily, member 10b ("TRAIL R2"), TRANCE, Troponin I, Urokinase Plasminogen Activator ("uPA"), Cadherin 5, type 2 or VE-cadherin (vascular endothelial) also known as CD144 ("VE-Cadherin"), WNTl-inducible-signaling pathway protein 1 ("WISP-1"), and Receptor Activator of Nuclear Factor κ B ("RANK").

In some implementations, the subject is also administered an immune checkpoint inhibitor. Immune Checkpoint inhibition broadly refers to inhibiting the checkpoints that cancer cells can produce to prevent or downregulate an immune response. Examples of immune checkpoint proteins include, but are not limited to, CTLA4, PD-1, PD-L1, PD-L2, A2AR, B7-H3, B7-H4, BTLA, KIR, LAG3, TIM-3 or VISTA. Immune checkpoint inhibitors can be antibodies or antigen binding fragments thereof that bind to and inhibit an immune checkpoint protein. Examples of immune checkpoint inhibitors include, but are not limited to, nivolumab, pembrolizumab, pidilizumab, AMP-224, AMP-514, STI-A1110, TSR-042, RG-7446, BMS-936559, MEDI-4736, MSB-0020718C, AUR-012 and STI-A1010.

In some implementations, a composition provided herein (*e.g.,* a vaccine composition provided herein) is administered prophylactically to prevent cancer, precancerous lesions, and/or an HPV infection. In some implementations, the vaccine is administered to inhibit tumor cell expansion. The vaccine may be administered prior to or after the detection of cancer cells, precancerous lesions, or HPV infected cells in a patient. Inhibition of tumor cell expansion is understood to refer to preventing, stopping, slowing the growth, or killing of tumor cells. In some implementations, after administration of a vaccine comprising peptides, nucleic acids, antibodies or APCs described herein, a proinflammatory response is induced. The proinflammatory immune response comprises production of proinflammatory cytokines and/or chemokines, for example, interferon gamma (IFN-γ) and/or interleukin 2 (IL-2). Proinflammatory cytokines and chemokines are well known in the art.

Conjunctive therapy includes sequential, simultaneous and separate, and/or co-administration of the active compounds in such a way that the therapeutic effects of the first agent administered have not entirely disappeared when the subsequent treatment is administered. In some implementations, the second agent may be co-formulated with the first agent or be formulated in a separate pharmaceutical composition.

Actual dosage levels of the active ingredients in the pharmaceutical compositions provided herein may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular agent employed, the route of administration, the time of administration, the rate of excretion or metabolism of the particular compound being employed, the duration of the treatment, other drugs, compounds and/or materials used in combination with the particular compound employed, the age, sex, weight, condition, general health and prior medical history of the patient being treated, and like factors well known in the medical arts.

In some aspects, provided herein is a method of identifying a subject suitable for a therapy provided herein (methods of treating an HPV infection, a cancer, and/or precancerous lesions in a subject comprising administering to the subject a pharmaceutical composition provided herein). In some implementations, the method comprises isolating a sample from the subject (*e.g.,* a blood sample, a tissue sample, a tumor sample) and detecting the presence of an HPV epitope listed in Table 1 in the sample. In some implementations the epitope is detected using an ELISA assay, a western blot assay, a FACS assay, a fluorescent microscopy assay, an Edman degradation assay and/or a mass spectrometry assay (e.g., protein sequencing). In some implementations, the presence of the HPV epitope is detected by detecting a nucleic acid encoding the HPV epitope. In some implementations, the nucleic acid encoding the HPV epitope is detected using a nucleic acid probe, a nucleic acid amplification assay and/or a sequencing assay.

Examples of nucleic acid amplification assays that can be used in the methods provided herein include, but are not limited to polymerase chain reaction (PCR), LATE-PCR, ligase chain reaction (LCR), strand displacement amplification (SDA), transcription mediated amplification (TMA), self-sustained sequence replication (3SR), Qβ replicase based amplification, nucleic acid sequence-based amplification (NASBA), repair chain reaction (RCR), boomerang DNA amplification (BDA) and/or rolling circle amplification (RCA).

In some implementations the product of the amplification reaction is detected as an indication of the presence and/or identity of the bacteria in the sample. In some implementations, the amplification product is detected after completion of the amplification reaction (*i.e.,* endpoint detection). Examples of end-point detection methods include gel-electrophoresis based methods, probe-binding based methods (*e.g*., molecular beacons, HPA probes, lights-on/lights-off probes) and double-stranded DNA binding fluorescent-dye based methods (e.g., ethidium bromide, SYBR-green). In some implementations, the amplification product is detected as it is produced in the amplification reaction (*i.e.,* real-time detection). Examples of real-time detection methods include probe-binding based methods (e.g., molecular beacons, TaqMan probes, scorpion probes, lights-on/lights-off probes) and double-stranded DNA binding fluorescent-dye based methods (*e.g.*, ethidium bromide, SYBR-green). In some implementations, the product of the amplification reaction is detected and/or identified by sequencing (*e.g*., through the use of a sequencing assay described herein).

In some implementations, the detection of the nucleic acid sequence comprises contacting the nucleic acid sequence with a nucleic acid probe that hybridizes specifically to the nucleic acid sequence. In some implementations, the probe is detectably labeled. In some implementations, the probe is labeled (directly or indirectly) with a fluorescent moiety. Examples of fluorescent moieties useful in the methods provided herein include, but are not limited to Allophycocyanin, Fluorescein, Phycoerythrin, Peridinin-chlorophyll protein complex, Alexa Fluor 350, Alexa Fluor 405, Alexa Fluor 430, Alexa Fluor 488, Alexa Fluor 514, Alexa Fluor 532, Alexa Fluor 546, Alexa Fluor 555, Alexa Fluor 568, Alexa Fluor 594, Alexa Fluor 633, Alexa Fluor 635, Alexa Fluor 647, Alexa Fluor 660, Alexa Fluor 680, Alexa Fluor 700, Alexa Fluor 750, Alexa Fluor 790, GFP, RFP, YFP, EGFP, mPlum, mCherry, mOrange, mKO, EYFP, mCitrine, Venus, YPet, Emerald, Cerulean and CyPet. In some implementations, the probe is a molecular beacon probe, a molecular torch probe, a TaqMan probes, a SDA probe, a scorpion probe, a HPA probe, or a lights on/lights off probe.

In some implementations, the nucleic acid sequence is detected by sequencing (e.g., whole genome sequencing, transcriptome sequence and/or targeted gene sequencing). Examples of sequencing processes that can be used in the methods provided herein include, but are not limited to, chain termination sequencing, massively parallel signature sequencing, ion semiconductor sequencing, polony sequencing, illumina sequencing, sequencing by ligation, sequencing by synthesis, pyrosequencing, single-molecule real-time sequencing, SOLiD sequencing, DNA nanoball sequencing, heliscope single molecule sequencing, single molecule real time sequencing, 454 sequencing, nanopore sequencing, tunneling currents DNA sequencing or sequencing by hybridization.

In some implementations, the methods provided herein further comprise treating the identified subject using a therapeutic method provided herein (*e.g.,* by administering to the subject a pharmaceutical composition provided herein).

### EXAMPLES

### Example 1: Characterizing the HPV-specific T cell response to facilitate the development of immunotherapeutic strategies for HPV

A total of 50 volunteers are recruited for this study. This includes patients who are yet to receive treatment, are currently being treated, or who have completed definitive radiation therapy ± systemic therapy for an HPV-associated oropharyngeal cancer. In addition to the patient cohort, up to 21 healthy donors are recruited for this study. An initial blood sample of up to 70 mL is collected from each participant - approximately 20 mL for HLA typing, and approximately 50 mL is collected into EDTA tubes for immunological assays. Three additional blood samples (50 mL each) is taken from participants to conduct follow-up studies on HPV immunity (especially in patients who have been diagnosed with HPV-positive cancer). If the participant is required to undergo surgery after the time of consent, fresh cancerous or pre-cancerous lesion specimens are collected at the time of surgery. In cases where sufficient tissue is available following the removal of a specimen for standard pathology analysis, a sample is investigated for histopathology and immunological studies.

HPV-specific T cell phenotype and function is assessed ex vivo, or following short-term in vitro culture. Studies include, but not be limited to: cell surface staining, peptide-MHC multimer analysis, ELISPOT, and intracellular cytokine staining assays. Gene expression and epigenetic analyses are undertaken, to assess the molecular regulation of effector function and phenotype in virus-specific T cells of patients compared with healthy donors, or virus-specific T cells compared with non-virus-specific T cells.

Total DNA is extracted from participant cells or plasma, and then quantitative real-time PCR is used to assess the HPV DNA load in these samples. Where available, cells from HPV-associated lesions are used to assess the immune response and antigen expression within the tissue, and also the expression of certain proteins and receptors that may modulate the immune response.

### Example 2: CD8⁺ and CD4⁺ T cell responses to HPV16 and HPV18 antigens

Virus-specific T-cells were expanded, in vitro, from PBMC taken from HNC (head and neck cancer). Briefly, PBMC samples were incubated with HPV antigen pepmix peptide pools for either HPV16 or HPV18 and cultured these cells for 14 days in the presence of IL-2, as outlined in each of Figures 1 and 2 respectively.

On day 14, these T cell cultures were assessed for HPV antigen-specificity using (Intracellular cytokine staining) ICS assay. The cut-off value for a positive response was taken to be 1% and applied to each of the identified IFNγ⁺CD8⁺ and IFNγ⁺CD4⁺ T cell populations. As seen in Figure 1, a dominant CD8⁺ response (i.e., IFNγ⁺) was observed for all HPV16 antigens, with the exception of E4. Similarly, for the CD4⁺ population, a dominant response was observed for all but the E4 and E5 HPV antigens. On the other hand, Figure 2 illustrates the low level of HPV18 antigen specific T cell response observed in HNC patients.

### Example 3: HPV T-cell epitope mapping

Peripheral blood mononuclear cells (PBMCs) from HNC patients were isolated and stimulated with HPV antigen pepmix peptide pools for either HPV16 or HPV18 in separate cultures and prepared for the intracellular cytokine staining (ICS) assay, as described above (non-stimulated controls were run in parallel). T cell response to the pepmix peptide pools was analyzed to identify individual peptides. These individual peptides were further assessed for T cell expansion and ICS analysis to identify potential epitopes. Once the 15mer peptide was identified, further mimimalization of the epitope sequence was carried out to identify the optimal T cell epitope sequence. The 15mer peptide sequences were trimmed from both N- and C-terminus to a minimal of 9-14 amino acid long peptides. Once the minimal peptide sequence was identified, further confirmation was carried out using limiting dose titration ICS assay. After mapping minimal epitope sequence, the HLA restriction of the epitope was identified by stimulating T cells using peptide loaded HLA-matched and mismatched PHA blasts (PHA). The complete process of epitope mapping is shown in the flowchart provided in Figure 3.

Representative data showing that HPV-specific T cells from HNC patients recognized HPV16-E6, HPV18-E6 antigens can be found in Figures 1 and 2, respectively. In order to map the T cell epitope, further analysis was carried out using sub pools of E6 peptides. For Example, with respect to HPV18-E6 peptides, pools E6-2, E6-3, and E6-9 were shown to be stimulatory via intracellular cytokine analysis. When analyzed on an HPV peptide matrix layout, the common peptide sequence among the responding pools was identified. (See Figure 4) The fine-mapping process identified TVLELTEVFEFA as the HPV18-E6 CD⁺ T cell epitope. (See Figure 5) Similarly, fine CD4 epitope-mapping of HPV16-E6 pools identified KQRFHNIRGRWTGRC. (See Figure 6) The list of HPV epitopes mapped during this study and in this manner are listed in Table 1.

### Example 4: HPV CD8 and CD4 polyepitope sequences

A series of HPV polyepitope peptides were designed. CD8⁺ T cell epitopes were selected (see Table 1) to generate an HPV CD8-polyepitope. (See Figure 7, A, individual epitopes are indicated by alternating bold and underlined italic text). Similarly, CD4⁺ T cell epitopes were selected from Table 1 to generate an HPV CD4-polyepitope. The CD4⁺ polyepitope includes an ER (endoplasmic reticulum) signal sequence at the amino-terminus and a lysosomal signal sequence at the carboxy-terminus. (See Figure 7, B). A polyepitope peptide construct was also designed to express both CD8⁺ and CD4⁺ T cell epitopes. In this case, the polyepitope sequences were separated by an internal ribosome entry site (IRES; derived from encephalomyocarditis virus). (See Figure 7, C and D) DNA sequence encoding HPV CD4⁺, CD8⁺ or CD8IRESCD4 polyepitopes were obtained from Atum Bio in a cloning pJ201 vector. Nucleotide sequences were released from cloning vector by digesting with 5' *Nhe* 1 and 3' *Kpn* 1 (for CD4⁺ and CD8⁺ polyepitopes) and 5' *Nhe* 1 and 3' *Not* 1 (for CD8IRESCD4poly) restriction enzymes. These poleypitope nucleotide sequences were cloned into pShuttle2 vector to make a mammalian expression cassette. Recombinant pShuttle2 vector was digested with I*-Ceu* I and PI*-Sce* I restriction enzymes to release mammalian expression cassette encoding HPV CD4⁺, CD8⁺ or CD8IRESCD4 polyepitopes and then ligated into Ad5F35 vector digested with I*-Ceu* I and PI*-Sce* I restriction enzymes. (See Figure 8) All the recombinant adenoviral vectors expressing HPV CD4⁺, CD8⁺ or CD8IRESCD4 polyepitopes were PCR confirmed and then vectors were linearized with *Pac* I restriction enzyme to expose the inverted terminal repeats for packaging of Ad5F35 DNA. To produce the recombinant adenovirus, HEK293 cells were transfected with linearised DNA and the primary recombinant virus stock was harvested on day 6 by lysing HEK293 cells with freeze-thawed cycles. To obtain the high titers of recombinant adenovirus, HEK293 cells were repeatedly infected with primary recombinant virus stock. The presence of HPV CD4⁺, CD8⁺ or CD8IRESCD4 polyepitopes nucleotide sequences in encapsidated adenoviral genome was evaluated by PCR (See Figure 9).

### Example 5: Evaluation of immunogenicity of recombinant AdHPVCD8

To investigate the immunogenicity of the AdHPVCD8poly, cells were pulsed with AdHPVCD8poly recombinant virus for one hour. Cells were then washed, incubated overnight, and then activation of a panel of HPV-specific T cells was assessed by measuring intracellular expression of IFN-γ. Representative data presented in Figure 10 clearly shows that HPV16-E2 HLA A02:01 restricted epitopes from HPV16-E2 and E6 antigens, and HLA A01:01 restricted epitope from HPV16-E2 antigen were more efficiently processed and presented to HPV-specific CD8⁺ T cells. The frequency of the HPV-specific CD8⁺ were similar to the JuSt fibroblasts pulsed with HPV pepmix.

### Example 6: Expansion of HPV-specific CD8⁺ T cells from HPV HNC patients using recombinant AdHPVCD8

To further evaluate the immunogenicity of the AdHPVCD8poly, PBMC from HPV HNC patients was stimulated with virus for 14 days in the presence of IL-2. The expansion of HPV-specific CD8⁺ T cells was then assessed by measuring IFN-γ secretion. Representative data presented in the Figure 11 shows that the HPV AdCD8poly induced expansion of HPV-specific CD8⁺ T cells in majority of the patients and expansion was observed against multiple antigens (E2, E4, E5, E6 and E7) from two different strains of HPV (HPV16 and HPV18).

### Example 7: Evaluation of immunogenicity of AdHPVCD4poly

To investigate the immunogenicity of the HPV AdCD4poly, PBMC from one of the HPV HNC patient was stimulated with AdHPVCD4poly and then cultured for 14 days in the presence of IL-2. Data presented in Figure 12 shows the robust expansion of HPV-specific CD4⁺ T cells following PBMC stimulation with HPV AdCD4poly compared to the PBMC stimulated with HPV pepmix and expansion of T cells directed toward multiple HPV16 E2, E6 and E7 antigens. Interestingly, as a possibility of incorporation of CD8⁺ T cell epitopes within the longer CD4⁺ T cell epitope, HPV AdCD4poly simultaneously stimulated expansion of HPV specific CD8⁺ T cells against HPV16 E6 and E7 antigens and the frequency of these CD8⁺ T cells were dramatically higher than PBMC stimulated with HPV pepmix (Figure 12).

### Example 8: Evaluation of immunogenicity of AdCD8IRESCD4 recombinant virus

To evaluate the immunogenicity of the HPV AdCD8IRESCD4poly, PBMC from 6 different HPV HNC patients were stimulated with the virus for 14 days in the presence of IL-2. Another set of the PBMC from the same patients was stimulated with AdHPVCD4poly and AdHPVCD8poly to compare the ability of AdCD8IRESCD4poly to expand HPV-specific CD4⁺ and CD8⁺ T cells. The expansion of HPV-specific CD4⁺ and CD8⁺ T cells were determined by measuring IFN-γ secretion. Representative data presented in Figures 13-A and -B shows that AdCD8IRESCD4poly induced expansion of HPV-specific CD4⁺ T cells against HPV16 E1, E6 and E7 from two donors and the frequency of the expanded T cells did not showed clear pattern compared to PBMC expanded with HPV AdCD4poly and HPV AdCD8poly. In addition, data presented in Figures 13-C and -D demonstrates that AdCD8IRESCD4poly also triggered expansion of HPV-specific CD8⁺ T cells from multiple donors against HPV16 E5, E6 and E7 antigens, which indicates capability of the HPV AdCD8IRESCD4poly as a single recombinant virus to expand HPV-specific CD4⁺ and CD8⁺ T cells from HPV HNC patients.

## Claims

1. A vaccine comprising a viral vector containing the CD8polyIRESCD4poly sequence of 
ATGTTCGAGCTGTCGCAAATGGTGCAGTGGACCCTGCTGCAACAGTACTGC
CTGTACTTGTCCGAGATCGCTTACAAATACGCCCAGCGGCCCTTTAAGAGC
AACAAGTCAACCACCCTGCAAGACGTCAGCCTCGAAGTGTACCTCGTGTGG
GAAGTCCACGCGGGTGGACAGGTCATCTGGCCCACAACTCCTCCGAGGCCA
ATCAATCTGGATACGGCGTCCACTACCCTCTCCGCCTTCCGGTGCTTCATCG
TGTACTCCCCTGCCACTGCCTTCACCGTGTATCACGACATTATTCTGGAATG
CGTGTACAAGCTCCCGCAGCTTTGTACCGAGCTGTTCCGCGATCTGTGCAT
CGTGTACCGCATTCGGTGCATCAACTGTCAGAAGCCGCTGACTGTGCTGGA
GCTGACCGAAGTGTTTGAGTTCGCCCTGTTGATGGGCACCCTTGGGATTGT
GGACAGAGCACATTACAACATCGTCACCTTCTGATAATAAGGTCTCTAAAAT
TCCGCCCCCCCCCTAACGTTACTGGCCGAAGCCGCTTGGAATAAGGCCGGTGTG
CGTTTGTCTATATGTTATTTTCCACCATATTGCCGTCTTTTGGCAATGTGAGGGCC
CGGAAACCTGGCCCTGTCTTCTTGACGAGCATTCCTAGGGGTCTTTCCCCTCTCG
CCAAAGGAATGCAAGGTCTGTTGAATGTCGTGAAGGAAGCAGTTCCTCTGGAAG
CTTCTTGAAGACAAACAACGTCTGTAGCGACCCTTTGCAGGCAGCGGAACCCCC
CACCTGGCGACAGGTGCCTCTGCGGCCAAAAGCCACGTGTATAAGATACACCTG
CAAAGGCGGCACAACCCCAGTGCCACGTTGTGAGTTGGATAGTTGTGGAAAGAG
TCAAATGGCTCTCCTCAAGCGTATTCAACAAGGGGCTGAAGGATGCCCAGAAGG
TACCCCATTGTATGGGATCTGATCTGGGGCCTCGGTACACATGCTTTACATGTGT
TTAGTCGAGGTTAAAAAACGTCTAGGCCCCCCGAACCACGGGGACGTGGTTTTC
CTTTGAAAAACACGATGATAATATGGCCACAACCATGCGCTACATGATTCTGGGG
CTCCTTGCACTCGCGGCCGTGTGCTCAGCCTGGAAGTCCTTTTTCTCCCGGACTTGGT
CGAGACTGTCACTCCATCATATCGACTACTGGAAGCACATGCGGCCTGGAATGCGGCCCT
GTACTCGGTGGATTCCGGCCCCGATCCTGACCGCTTTCAACTCCTCCCACGTCTATGAC
TACGGCCTTCCGGGACCTCTGTATTGTCTACCGGGATGGGAACCCGTACGCCGTGTGT
GACCGGTGCATCAATTGCCAGAAGCCACTCTGCCCTGAGGAAAAGCAGAGGCATCTG
GACGAGAAGCAACGCCATTTGGATAAGAAACAGCGGTTCCACAACATCAGAGGTCGC
TGGACTGGCCGGTGCATGTCCTGCTGCACCCCCACCCTGCACGAGTACATGCTGGAT
CTGCAGCCCGAGACTACCGACCTGTACTACGAACAGCTTAACGACAGCTCCGAGGAA
GAGGACGAAATCGACGTGCAAAGCACCCACGTGGACATCAGGACCCTGGAAGATCTC
CTGATGGGCACCCTGACGTTGATCCCTATTGCTGTCGGTGGAGCACTTGCCCGGCCTG
GTGCTGATCGTGCTGATCGCCTATCTCGTGGGACGCAAGAGAAGCCACGCGGGATAC
CAGACTATTTGATAA
 HPV CD8poly (in bold)
IRES (underlined)
*HPV CD4poly* (in italic)
encoding a polyepitope peptide comprising Human Papillomavirus (HPV) peptide epitopes capable of inducing proliferation of peptide-specific T cells, wherein the viral vector is a recombinant adenovirus Ad5F35, and a pharmaceutically acceptable carrier, diluent or excipient.

2. The vaccine of claim 1, wherein the peptide epitopes are capable of inducing proliferation of peptide-specific cytotoxic T cells (CTLs).

3. The vaccine of claim 1 or claim 2 for use in treating and/or preventing a cancer expressing an HPV epitope or a precancerous condition expressing an HPV epitope in a subject.

4. The vaccine of claim 1 or claim 2 for use in treating and/or preventing an HPV infection in a subject.

## Patentansprüche

1. Impfstoff, umfassend einen viralen Vektor, der die CD8polyIRESCD4poly-Sequenz von 
ATGTTCGAGCTGTCGCAAATGGTGCAGTGGACCCTGCTGCAACAGTACTGC
CTGTACTTGTCCGAGATCGCTTACAAATACGCCCAGCGGCCCTTTAAGAGC
AACAAGTCAACCACCCTGCAAGACGTCAGCCTCGAAGTGTACCTCGTGTGG
GAAGTCCACGCGGGTGGACAGGTCATCTGGCCCACAACTCCTCCGAGGCCA
ATCAATCTGGATACGGCGTCCACTACCCTCTCCGCCTTCCGGTGCTTCATCG
TGTACTCCCCTGCCACTGCCTTCACCGTGTATCACGACATTATTCTGGAATG
CGTGTACAAGCTCCCGCAGCTTTGTACCGAGCTGTTCCGCGATCTGTGCAT
CGTGTACCGCATTCGGTGCATCAACTGTCAGAAGCCGCTGACTGTGCTGGA
GCTGACCGAAGTGTTTGAGTTCGCCCTGTTGATGGGCACCCTTGGGATTGT
GGACAGAGCACATTACAACATCGTCACCTTCTGATAATAAGGTCTCTAAAAT
TCCGCCCCCCCCCTAACGTTACTGGCCGAAGCCGCTTGGAATAAGGCCGGTGTG
CGTTTGTCTATATGTTATTTTCCACCATATTGCCGTCTTTTGGCAATGTGAGGGCC
CGGAAACCTGGCCCTGTCTTCTTGACGAGCATTCCTAGGGGTCTTTCCCCTCTCG
CCAAAGGAATGCAAGGTCTGTTGAATGTCGTGAAGGAAGCAGTTCCTCTGGAAG
CTTCTTGAAGACAAACAACGTCTGTAGCGACCCTTTGCAGGCAGCGGAACCCCC
CACCTGGCGACAGGTGCCTCTGCGGCCAAAAGCCACGTGTATAAGATACACCTG
CAAAGGCGGCACAACCCCAGTGCCACGTTGTGAGTTGGATAGTTGTGGAAAGAG
TCAAATGGCTCTCCTCAAGCGTATTCAACAAGGGGCTGAAGGATGCCCAGAAGG
TACCCCATTGTATGGGATCTGATCTGGGGCCTCGGTACACATGCTTTACATGTGT
TTAGTCGAGGTTAAAAAACGTCTAGGCCCCCCGAACCACGGGGACGTGGTTTTC
CTTTGAAAAACACGATGATAATATGGCCACAACCATGCGCTACATGATTCTGGGG
CTCCTTGCACTCGCGGCCGTGTGCTCAGCCTGGAAGTCCTTTTTCTCCCGGACTTGGT
CGAGACTGTCACTCCATCATATCGACTACTGGAAGCACATGCGCCTGGAATGCGCCCT
GTACTCGGTGGATTCCGCCCCGATCCTGACCGCTTTCAACTCCTCCCACGTCTATGAC
TACGCCTTCCGGGACCTCTGTATTGTCTACCGGGATGGGAACCCGTACGCCGTGTGT
GACCGGTGCATCAATTGCCAGAAGCCACTCTGCCCTGAGGAAAAGCAGAGGCATCTG
GACGAGAAGCAACGCCATTTGGATAAGAAACAGCGGTTCCACAACATCAGAGGTCGC
TGGACTGGCCGGTGCATGTCCTGCTGCACCCCCACCCTGCACGAGTACATGCTGGAT
CTGCAGCCCGAGACTACCGACCTGTACTACGAACAGCTTAACGACAGCTCCGAGGAA
GAGGACGAAATCGACGTGCAAGCACCCACGTGGACATCAGGACCCTGGAAGATCTC
CTGATGGGCACCCTGACGTTGATCCCTATTGCTGTCGGTGGAGCACTTGCCGGCCTG
GTGCTGATCGTGCTGATCGCCTATCTCGTGGGACGCAAGAGAAGCCACGCGGGATAC
CAGACTATTTGATAA
 HPV CD8poly (fettgedruckt)
IRES (unterstrichen)
*HPV CD4poly* (kursiv)
enthält, die ein Polyepitop-Peptid kodiert, das Humane Papillomvirus (HPV)-Peptidepitope umfasst, die in der Lage sind, die Proliferation von peptidspezifischen T-Zellen zu induzieren, wobei der virale Vektor ein rekombinanter Adenovirus Ad5F35 ist, und einen pharmazeutisch akzeptablen Träger, ein Verdünnungsmittel oder einen Hilfsstoff.

2. Impfstoff nach Anspruch 1, wobei die Peptidepitope in der Lage sind, die Proliferation von peptidspezifischen zytotoxischen T-Zellen (CTLs) zu induzieren.

3. Impfstoff nach Anspruch 1 oder Anspruch 2 zur Verwendung bei der Behandlung und/oder Prävention eines Krebses, der ein HPV-Epitop exprimiert, oder einer präkanzerösen Erkrankung, die ein HPV-Epitop exprimiert, bei einem Individuum.

4. Impfstoff nach Anspruch 1 oder Anspruch 2 zur Verwendung bei der Behandlung und/oder Prävention einer HPV-Infektion bei einem Individuum.

## Revendications

1. Vaccin comprenant un vecteur viral contenant la séquence de CD8polyIRESCD4poly suivante : 
ATGTTCGAGCTGTCGCAAATGGTGCAGTGGACCCTGCTGCAACAGTACTGC
CTGTACTTGTCCGAGATCGCTTACAAATACGCCCAGCGGCCCTTTAAGAGC
AACAAGTCAACCACCCTGCAAGACGTCAGCCTCGAAGTGTACCTCGTGTGG
GAAGTCCACGCGGGTGGACAGGTCATCTGGCCCACAACTCCTCCGAGGCCA
ATCAATCTGGATACGGCGTCCACTACCCTCTCCGCCTTCCGGTGCTTCATCG
TGTACTCCCCTGCCACTGCCTTCACCGTGTATCACGACATTATTCTGGAATG
CGTGTACAAGCTCCCGCAGCTTTGTACCGAGCTGTTCCGCGATCTGTGCAT
CGTGTACCGCATTCGGTGCATCAACTGTCAGAAGCCGCTGACTGTGCTGGA
GCTGACCGAAGTGTTTGAGTTCGCCCTGTTGATGGGCACCCTTGGGATTGT
GGACAGAGCACATTACAACATCGTCACCTTCTGATAATAAGGTCTCTAAAAT
TCCGCCCCCCCCCCTAACGTTACTGGCCGAAGCCGCTTGGAATAAGGCCGGTGTG
CGTTTGTCTATATGTTATTTTCCACCATATTGCCGTCTTTTGGCAATGTGAGGGCC
CGGAAACCTGGCCCTGTCTTCTTGACGAGCATTCCTAGGGGTCTTTCCCCTCTCG
CCAAAGGAATGCAAGGTCTGTTGAATGTCGTGAAGGAAGCAGTTCCTCTGGAAG
CTTCTTGAAGACAAACAACGTCTGTAGCGACCCTTTGCAGGCAGCGGAACCCCC
CACCTGGCGACAGGTGCCTCTGCGGCCAAAAGCCACGTGTATAAGATACACCTG
CAAAGGCGGCACAACCCCAGTGCCACGTTGTGAGTTGGATAGTTGTGGAAAGAG
TCAAATGGCTCTCCTCAAGCGTATTCAACAAGGGGCTGAAGGATGCCCAGAAGG
TACCCCATTGTATGGGATCTGATCTGGGGCCTCGGTACACATGCTTTACATGTGT
TTAGTCGAGGTTAAAAAACGTCTAGGCCCCCCGAACCACGGGGACGTGGTTTTC
CTTTGAAAAACACGATGATAATATGGCCACAACCATGCGCTACATGATTCTGGGG
CTCCTTGCACTCGCGGCCGTGTGCTCAGCCTGGAAGTCCTTTTTCTCCCGGACTTGGT
CGAGACTGTCACTCCATCATATCGACTACTGGAAGCACATGCGCCTGGAATGCGCCCT
GTACTCGGTGGATTCCGCCCCGATCCTGACCGCTTTCAACTCCTCCCACGTCTATGAC
TACGCCTTCCGGGACCTCTGTATTGTCTACCGGGATGGGAACCCGTACGCCGTGTGT
GACCGGTGCATCAATTGCCAGAAGCCACTCTGCCCTGAGGAAAGCAGAGGCATCTG
GACGAGAAGCAACGCCATTTGGATAAGAAACAGCGGTTCCACAACATCAGAGGTCGC
TGGACTGGCCGGTGCATGTCCTGCTGCACCCCCACCCTGCACGAGTACATGCTGGAT
CTGCAGCCCGAGACTACCGACCTGTACTACGAACAGCTTAACGACAGCTCCGAGGAA
GAGGACGAAATCGACGTGCAAAGCACCCACGTGGACATCAGGACCCTGGAAGATCTC
CTGATGGGCACCCTGACGTTGATCCCTATTGCTGTCGGTGGAGCACTTGCCGGCCTG
GTGCTGATCGTGCTGATCGCCTATCTCGTGGGACGCAAGAGAAGCCACGCGGGATAC
CAGACTATTTGATAA
 HPV CD8poly (en gras)
IRES (souligné)
*HPV CD4poly* (en italique)
codant un peptide polyépitopique comprenant des épitopes de peptide de papillomavirus humain (HPV) capables d'induire une prolifération de cellules T spécifiques du peptide, dans lequel le vecteur viral est un adénovirus Ad5F35 recombiné, et un véhicule, diluant ou excipient pharmaceutiquement acceptable.

2. Vaccin selon la revendication 1, dans lequel les épitopes de peptide sont capables d'induire une prolifération de cellules T cytotoxiques (CTL) spécifiques du peptide.

3. Vaccin selon la revendication 1 ou la revendication 2, pour une utilisation dans le traitement et/ou la prévention d'un cancer exprimant un épitope de HPV ou un état précancéreux exprimant un épitope de HPV chez un sujet.

4. Vaccin selon la revendication 1 ou la revendication 2, pour une utilisation dans le traitement et/ou la prévention d'une infection par HPV chez un sujet.
